(19) Europäisches Patentamt
European Patent Office
Office européen des brevets

(11) Veröffentlichungsnummer: **0 175 363**
**B1**

(12) # EUROPÄISCHE PATENTSCHRIFT

(45) Veröffentlichungstag der Patentschrift: **17.01.90**

(21) Anmeldenummer: **85111838.0**

(22) Anmeldetag: **19.09.85**

(51) Int. Cl.[4]: **C 07 D 237/04**, C 07 D 403/10, C 07 D 403/12, C 07 D 403/04, C 07 D 401/10, C 07 D 401/12, C 07 D 417/12, C 07 D 405/14, C 07 D 413/10, C 07 D 413/12, A 61 K 31/50

(54) **4, 5-Dihydro-3(2H)-pyridazinone, Verfahren zu ihrer Herstellung und ihre Verwendung.**

(30) Priorität: **21.09.84 DE 3434680**
**21.06.85 DE 3522193**

(43) Veröffentlichungstag der Anmeldung:
**26.03.86 Patentblatt 86/13**

(45) Bekanntmachung des Hinweises auf die Patenterteilung:
**17.01.90 Patentblatt 90/03**

(84) Benannte Vertragsstaaten:
**AT BE CH DE FR GB IT LI LU NL SE**

(56) Entgegenhaltungen:
**EP-A-0 117 403**
**BE-A-871 310**
**DE-A-2 151 216**
**DE-A-2 157 453**

**PATENTS ABSTRACTS OF JAPAN, Band 7, Nr. 77 (C-159) 1222 , 30. März 1983; & JP-A-58 8016**

**Medicinal Chemistry, 3 Auflage, S. 74-77**

(73) Patentinhaber: **CASSELLA Aktiengesellschaft**
**Hanauer Landstrasse 526**
**D-6000 Frankfurt am Main 61 (DE)**

(72) Erfinder: **Zoller, Gerhard, Dr.**
**Höhenstrasse 8**
**D-6369 Schöneck (DE)**
Erfinder: **Beyerle, Rudi, Dr.**
**An der Pfaffenmauer 44**
**D-6000 Frankfurt am Main (DE)**
Erfinder: **Just, Melitta, Dr.**
**Hüttenweg 6**
**D-6369 Schöneck (DE)**
Erfinder: **Bohn, Helmut, Dr.**
**Kranzbergring 11**
**D-6369 Schöneck (DE)**
Erfinder: **Martorana, Piero, Dr.**
**Kaiser-Friedrich-Promenade 108**
**D-6380 Bad Homburg (DE)**
Erfinder: **Nitz, Rolf-Eberhard, Dr.**
**Heinrich-Bingemer-Weg 64**
**D-6000 Frankfurt am Main (DE)**

(74) Vertreter: **Urbach, Hans-Georg, Dr.**
**Hanauer Landstrasse 526**
**D-6000 Frankfurt am Main 61 (DE)**

EP 0 175 363 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die vorliegende Erfindung betrifft substituierte 4,5-Dihydro-3(2H)-pyridazinone der Formel I

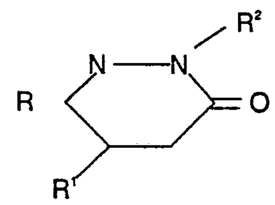

(I)

worin

R einen Rest der Formel

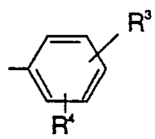

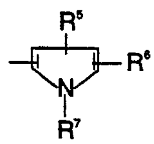

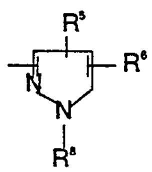

oder

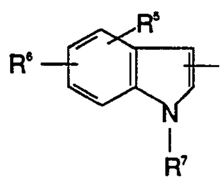

$R^1$, $R^2$ unabhängig voneinander Wasserstoff oder $(C_1$-$C_4)$Alkyl,

$R^3$ $(C_1$-$C_4)$Alkoxy-$(C_1$-$C_4)$alkoxy, Amino-carbonyl-$(C_1$-$C_4)$alkoxy, $(C_1$-$C_4)$Alkoxy-$(C_1$-$C_4)$alkyl-amino-carbonyl-$(C_1$-$C_4)$alkoxy, Thiazolidinyl-carbonyl-amino, Mono- $(C_1$-$C_4)$alkylamino-carbonyl-$(C_1$-$C_4)$alkoxy, $(C_1$-$C_4)$Alkoxy-carbonyl-$(C_1$-$C_4)$alkoxy, Hydroxy-carbonyl-$C_1$-$C_4)$alkoxy, $(C_1$-$C_4)$Alkyl-thio, $(C_1$-$C_4)$Alkyl-sulfinyl, $(C_1$-$C_4)$-Alkyl-sulfonyl, (2-Oxo-pyrrolidinyl)-$(C_1$-$C_4)$alkyl-amino-carbonyl-amino, (2-Oxo-piperidinyl)-$(C_1$-$C_4)$-alkyl-amino-carbonyl-amino, 2-Oxo-pyrrolidinyl, 2-Oxo-piperidinyl, 2,5-Dioxo-piperidinyl, 2,5-Dioxo-pyrrolidinyl, 2-Oxo-imidazolidinyl, 2-Oxo-hexahydro-pyrimidinyl, 2,4-Dioxo-imidazolidinyl, 2,4-Dioxo-hexahydro-pyrimidinyl, 2-Oxo-1,3-oxazolidinyl, 3-Oxo-pyrazolidinyl, (2-($R^9$-carbonyl)-pyrrolidinyl-$(C_1$-$C_4)$alkoxy, $(C_1$-$C_4)$Alkyl und $(C_1$-$C_4)$Alkoxy substituiert durch Pyridyl, Imidazolyl, Oxadiazolyl, Oxo-pyranyl, 2-Hydroxy-pyridinyl, Pyrrolinyl oder Oxo-oxazolidinyl, wobei das Oxo-pyranyl, Oxo-oxazolidinyl und Oxadiazolyl seinerseits durch $(C_1$-$C_4)$Alkyl oder $(C_1$-$C_4)$Alkoxy-carbonyl substituiert sein kann, oder einen Rest der Formel $R^{10}$-CO-NH-,

$R^4$ Wasserstoff, $(C_1$-$C_4)$Alkyl, $(C_1$-$C_4)$Alkoxy, Hydroxy, $(C_1$-$C_5)$Alkanoyloxy oder Halogen,

$R^5$, $R^6$, $R^7$ unabhängig voneinander Wasserstoff, $(C_1$-$C_4)$-Alkyl, $(C_1$-$C_4)$Alkoxy, Hydroxy, Halogen, Amino, Mono-$(C_1$-$C_4)$alkylamino oder Di-$(C_1$-$C_4)$alkylamino,

$R^8$ Wasserstoff, $(C_1$-$C_4)$Alkyl oder Phenyl,

$R^9$ Hydroxy, $(C_1$-$C_4)$Alkoxy, Amino, Mono-$(C_1$-$C_4)$alkylamino oder Di-$(C_1$-$C_4)$alkylamino,

$R^{10}$ p-Chlorphenoxymethyl, 3-Pyridyl-oxy-methyl, 3-Pyridyl-methoxy-methyl, 4-Pyridyl-thiomethyl, 4-Pyridyl-sulfinyl-methyl, 4-Pyridyl-sulfonyl-methyl, 2-Oxo-thiazolidin-4-yl, 3-Oxo-perhydro-1,4-thiazin-5-yl, 1-Oxido-3-oxo-perhydro-1,4-thiazin-5-yl, 5-Oxoperhydro-1,4-thiazepin-3-yl, 1-Oxido-5-oxo-perhydro-1,4-thiazepin-3-yl, 1,1-Dioxido-5-oxo-perhydro-1,4-thiazepin-3-yl, oder einen Rest der Formel

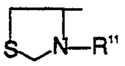 ; 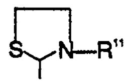

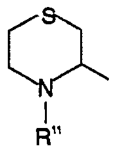 ; 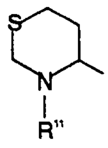

$R^{11}$ Wasserstoff oder einen Rest der Formel

$R^{12}$–CO–,

$R^{12}$ Wasserstoff, Alkyl mit 1 bis 5 C-Atomen, Alkoxy mit 1 bis 5 C-Atomen, Benzyl oder Benzyloxy bedeuten, und ihre pharmakologisch annehmbaren Additionssalze.

Die Erfindung betrifft auch Verfahren zur Herstellung der Verbindungen der Formel I und ihre Verwendung als pharmakologische Präparate.

Aus der BE-A-871 310 sind 4,5-Dihydro-3(2H)-pyridazinone mit antithrombotischen und antihypertensiven Eigenschaften bekannt, die in 6-Stellung durch über ihre 6-Stellung gebundene Tetrahydrochinolin-2-one oder Tetrahydrochinoline oder durch über ihre 5-Stellung gebundene Indolin-2-one oder Indoline substituiert sind.

Aus Patent Abstracts of Japan, Band 7, Nr.77 (JP-A-58/8016) sind 4,5-Dihydro-3(2H)-pyridazinone bekannt, die in 6-Stellung ein 2,3-Dihydro-indolin-5-yl, ein 1,2,3,4-Tetrahydrochinolin-6-yl oder einen Phenylrest tragen, der in para-Stellung z. B. durch Amino, ($C_1$-$C_6$)Alkylamino, Di($C_1$-$C_6$)alkylamino, ($C_1$-$C_6$)Acylamino oder N-($C_1$-$C_6$)Alkyl-N-($C_1$-$C_6$)acyl-amino substituiert ist. Die Verbindungen werden als cardiotonische Wirkstoffe verwendet.

Aus der EP-A-117 403 sind 6-(Acylaminophenyl)-4,5-dihydro-3(2H)-pyridazinonderivate bekannt, deren Acylaminorest durch Amino substituiert ist, wobei dieses Amino auch 1- oder 2-fach z. B. durch ($C_1$-$C_{12}$)Kohlenwasserstoffreste substituiert oder Teil eines gegebenenfalls substituierten heterocyclischen Rings sein kann. Diese Verbindungen sind Thrombozytenaggregationshemmer, Blutdrucksenker und Magensekretionshemmer.

Die erfindungsgemäßen Verbindungen werden durch die bekannten Verbindungen nicht nahegelegt.

Die für $R^1$ bis $R^{12}$ stehenden Alkyl- und/oder Alkoxyreste können, auch wenn sie in Kombination miteinander stehen bzw. als Substituenten oder gegenseitige Substituenten auftreten, geradkettig oder verzweigt sein.

Das für $R^4$, $R^5$, $R^6$ und $R^7$ stehende Halogen bedeutet insbesondere Brom oder Chlor.

Der für $R^4$ stehende Alkanoyloxyrest bedeutet z. B. Formyloxy, Acetoxy, Butyryloxy, Isobutyryloxy, Valeryloxy, Isovaleryloxy.

$R^1$ und $R^2$ bedeuten unabhängig voneinander vorzugsweise Wasserstoff und/oder Methyl. Besonders bevorzugt steht, $R^1$ für Methyl und $R^2$ für Wasserstoff.

Für R sind bevorzugt: der durch $R^3$ und $R^4$ substituierte Phenylrest, insbesondere der durch $R^3$ in 4-Stellung substituierte Phenylrest, Pyrrolyl, insbesondere 2-Pyrrolyl, Indolyl, insbesondere 3-Indolyl, 5-Amino-1,3-dimethyl-pyrazol-4-yl, 5-Hydroxy-3-methyl-1-phenyl-pyrazol-4-yl. Für R ist weiterhin der Rest

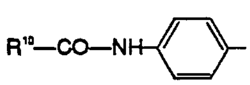

bevorzugt.

$R^4$ ist vorzugsweise Wasserstoff, ($C_1$-$C_4$)Alkyl oder ($C_1$-$C_4$)Alkoxy. $R^4$ ist insbesondere Wasserstoff oder Methoxy und ganz besonders bevorzugt Wasserstoff.

$R^3$ ist vorzugsweise: 2-Methoxy-ethoxy, 3-Pyridyl-methoxy, Amino-carbonyl-methoxy, Hydroxy-carbonyl-methoxy, Methylthio, (2-Methoxy-ethyl)-amino-carbonyl-methoxy, 3-Pyridyl-methyl, 5-Methyl-1,3,4-oxadiazol-2-yl, 2-Hydroxy-4-methyl-pyrid-6-yl-methoxy, 2-(Imidazol-1-yl)-ethoxy, (2-Oxo-oxazolidin-5-yl)-methoxy, 2-(2-Oxo-pyrrolidin-1-yl)-ethyl-amino-carbonyl-amino, 2-(Methoxycarbonyl-pyrrolidin-1-yl)-ethoxy, 2-Oxo-pyrrolidin-1-yl, 2,5-Dioxopyrrolidin-1-yl, 2-Oxo-imidazolidin-1-yl, 3-Oxo-pyrazolidin-1-yl, 2,4-Dioxo-imidazolidin-1-yl, 2-Oxo-oxazolidin-3-yl, Thiazolidin-4-yl-carbonyl-amino. $R^3$ ist weiterhin vorzugsweise ein Rest der Formel $R^{10}$-CO-NH-.

Beispiele für den Rest $R^{11}$ sind: Wasserstoff, Formyl, Acetyl, Propionyl, Butyryl, Isobutyryl, Valeryl, Isovaleryl, Pivaloyl, Phenylacetyl, Methoxy-carbonyl, Ethoxy-carbonyl, Propoxy-carbonyl, Isopropoxy-carbonyl, Butoxy-carbonyl, Isobutoxy-carbonyl, tert.-Butoxy-carbonyl, Benzyloxy-carbonyl. Für den Rest $R^{11}$ sind Wasserstoff, Formyl, Acetyl,

tert.-Butoxy-carbonyl und Benzyloxy-carbonyl bevorzugt.

Bevorzugte Verbindungen der Formel I besitzen eine oder vorzugsweise mehrere bevorzugte Reste für R bis $R^{12}$. Die Verbindungen der Formel I können in tautomeren Formen vorliegen, falls die Voraussetzungen hierfür gegeben sind.

Bevorzugte Verbindungen sind auch substituierte 4,5-Dihydro-3(2H)-pyridazinone der Formel Ia

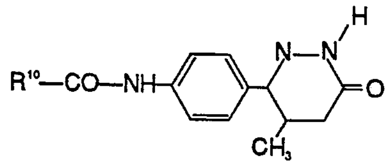

(Ia)

wobei $R^{10}$ die bereits genannte Bedeutung besitzt.

Die Verbindungen der Formel I lassen sich in Analogie zu der Herstellung anderer 4,5-Dihydro-3(2H)-pyridazinone dadurch herstellen, daß eine Carbonsäure oder ein Carbonsäurederivat der Formel II mit einem N-$R^2$-Hydrazin der Formel III nach folgender Reaktionsgleichung umgesetzt wird:

$$R{-}\overset{\displaystyle O}{\overset{\|}{C}}{-}\underset{\displaystyle R^1}{\underset{|}{CH}}{-}CH_2{-}X \quad + \quad H_2N{-}NHR^2 \longrightarrow \quad (I)$$

(II) (III)

In den Formeln II und III besitzen R, $R^1$ und $R^2$ die bereits genannten Bedeutungen. In Formel II bedeutet X = -COOH, -COCl, -CO-O-CO-$R^{13}$, -CO-O$R^{13}$ oder -CN, wobei $R^{13}$ ein organischer Rest, insbesondere ein Alkylrest, vorzugsweise ein Alkylrest mit 1 bis 4 C-Atomen, ganz besonders bevorzugt Methyl, bedeutet.

Bei $R^2$ = H handelt es sich bei der Verbindung der Formel III um das Hydrazin selbst. Bei $R^2$ = Alkyl handelt es sich bei der Verbindung III um ein asymmetrisch alkyliertes Hydrazin. Die Verbindung der Formel III kann auch in Form ihres Hydrats eingesetzt werden. Die Umsetzung zwischen den Verbindungen II und III wird zweckmäßigerweise in flüssiger Phase durchgeführt, wozu in der Regel die Anwesenheit eines inerten Lösungs- oder Verdünnungsmittels erforderlich ist.

Geeignete Lösungs- oder Verdünnungsmittel sind z. B. Alkohole, insbesondere solche mit 1 bis 6 C-Atomen, wie z. B. Methanol, Ethanol, i- und n-Propanol, i-, sec.- und tert.-Butanol, n-, i-, sec.-, tert.-Pentanol, n-Hexanol, Cyclopentanol, Cyclohexanol; Ether, insbesondere solche mit 2 bis 8 C-Atomen im Molekül, wie z.B. Diethylether, Methyl-ethyl-ether, Di-n-propyl-ether, Di-isopropyl-ether, Methyl-n-butyl-ether, Ethylpropyl-ether, Di-butyl-ether, Tetrahydrofuran; 1,4-Dioxan, 1,2-Dimethoxyethan, Bis-β-methoxyethyl-ether; Polyether, wie z. B. Polyethylenglykole mit einem Molekulargewicht bis ca 600; Oligoethylenglycol-dimethyl-ether, wie z. B. Pentaglyme; Kronenether, d.h. cyclische Polymere des Ethylenglykols der Formel $(-OCH_2CH_2)_p$, wobei p eine Zahl z. B. von 4 bis 10 ist, wobei an den Ring auch eine oder mehrere Benzolringe ankondensiert sein können; Aza- und Thia-kronenether (Coronand-amine und Coronand-sulfide); Glykole und teilweise veretherte Glykole, wie z. B. Ethylenglykol, Propylenglykol, Trimethylenglykol, Ethylenglykol-monomethyl-ether, Ethylenglykol-monoethyl-ether, Diethylenglykolmonoethyl-ether; aliphatische Kohlenwasserstoffe, wie z. B. Benzine, niedrig- und hochsiedende Petrolether; aromatische Kohlenwasserstoffe, wie z. B. Benzol, Toluol, o-, m- und p-Xylol, Pyridin; halogenierte aliphatische oder aromatische Kohlenwasserstoffe, wie z. B. Methylenchlorid, Chloroform, Tetrachlorkohlenstoff, Ethylenchlorid, Chlorbenzol, Dichlorbenzol; Nitrile, wie z. B. Acetonitril; Amide, wie z. B. Dimethylformamid, N-Methyl-pyrrolidon; Sulfoxide, wie z. B. Dimethyl-sulfoxid; Wasser. Auch Gemische verschiedener Lösungs- oder Verdünnungsmittel können verwendet werden.

Die Umsetzung zwischen den Verbindungen II und III kann prinzipiell bei Temperaturen zwischen Raumtemperatur und dem Siedepunkt des verwendeten Lösungs- oder Verdünnungsmittels erfolgen.

Die Verbindungen der Formel III sind bekannt oder lassen sich nach bekannten Verfahren für die Herstellung von N-Alkylhydrazinen synthetisieren. Die Verbindungen der Formel II lassen sich nach verschiedenen, an sich bekannten Verfahren herstellen, so z. B. durch Acylierung der Verbindungen R-H mit $R^1$-Maleinsäureanhydrid und anschließender Hydrierung der erhaltenen ungesättigten Verbindung, z. B. nach dem im J. Med. Chem. *17*, 273 ff., (1974), beschriebenen Verfahren, ferner durch Mannich-Reaktion von Ketonen der Formel $R{-}CO{-}CH_2{-}R^1$ mit Formaldehyd/Dimethylamin zu Mannich-Basen $R{-}CO{-}CH(R^1){-}CH_2{-}N(CH_3)_2$ und anschließendem Austausch des Aminrestes, z. B. über die quartäre Iodverbindung gegen Cyan, wobei Verbindungen $R{-}CO{-}CH(R^1){-}CH_2{-}CN$ und gewünschtenfalls hieraus durch Hydrolyse Verbindungen $R{-}CO{-}CH(R^1){-}CH_2{-}COOH$ erhalten werden. Diese Reaktionsfolge ist z. B. beschrieben in J. Org. Chem. *38*, 4044 ff., (1973). Die Verbindungen der Formel II mit X = CN lassen sich z. B. auch durch Acylierung von Verbindungen der Formel R-H mit 2-$R^1$-3-Chlor-propionsäurechlorid gemäß DE-OS-3 328 286 herstellen, wobei zunächst Verbindungen der Formel R-CO-CH($R^1$)-$CH_2$-Cl entstehen, an denen das Chlor z. B. mit NaCN oder KCN gegen CN ausgetauscht wird.

Verbindungen der Formel II mit X = CN liefern bei der Umsetzung mit den Verbindungen der Formel III direkt die Verbindungen der Formel I, wenn die Reaktion in wäßrigem Medium oder in Anwesenheit von Wasser durchgeführt wird. Bei der Durchführung der Reaktion in wasserfreiem Medium entstehen zunächst die Ketimine der Verbindungen der Formel I, die anschließend in an sich bekannter Weise zu den Verbindungen der Formel I hydrolysiert werden.

Ausgangsverbindungen der Formel II mit X = CN lassen sich leicht nach an sich bekannten Verfahren in Ausgangsverbindungen der Formel II mit anderen Bedeutungen von X umwandeln.

Ausgangsverbindungen der Formel II mit $R^1$ = -$CH_3$ und X = -CO-$OR^{13}$, vorzugsweise mit $R^{13}$ = -$CH_3$, lassen sich besonders einfach und in hoher Ausbeute dadurch herstellen, daß Verbindungen der Formel R-H mit 3-($R^{13}$O-Carbonyl)-isobutyryl-chlorid (= $R^{13}$O-CO-$CH_2$-CH($CH_3$)-CO-Cl) unter den Bedingungen der Ketonsynthese nach Friedel-Crafts acyliert werden.

Verbindungen der Formel I, bei denen $R^2$ einen Alkylrest bedeutet, können auch aus Verbindungen der Formel I, bei denen $R^1$ Wasserstoff bedeutet, durch Umsetzung in an sich bekannter Weise mit einem den Alkylrest $R^2$ einführenden Alkylierungsmittel hergestellt werden. Die Alkylierung wird zweckmäßigerweise in einem geeigneten Lösungsmittel bei Temperaturen von Raumtemperatur bis zur Rückflußtemperatur des Lösungsmittels durchgeführt. Als Alkylierungsmittel kommen z. B. Alkyhalogenide, Dialkylsulfate oder Alkyltosylate in Betracht, durch die der Alkylrest $R^2$ eingeführt wird.

Die Verbindungen der Formel Ia lassen sich in Analogie zur Herstellung anderer Carbonsäureamide durch Acylierung von 6-(4-Aminophenyl)-4,5-dihydro-5-methyl-3(2H)-pyridazinon der Formel IV

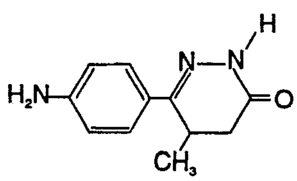

(IV)

mit Acylierungsmitteln, die den Acylrest $R^{10}$-CO- einführen, herstellen. Bei dieser Acylierung wird ein Wasserstoffatom der Aminogruppe der Verbindung II durch den Rest $R^{10}$-CO- ersetzt. Geeignete Acylierungsmittel sind z. B. Verbindungen der Formel V

$$R^{10}\text{–CO–Y} \quad \text{(V)}$$

worin $R^{10}$ die bereits genannte Bedeutung besitzt und Y = Halogen, insbesondere -Cl oder -Br, -OH, -O-Alkyl, insbesondere mit 1 bis 5 C-Atomen, -O-CO-$R^{10}$ oder -O-CO-O-Alkyl, insbesondere mit 1 bis 5 C-Atomen im Alkylrest, -O-Aryl, -O-Nitroaryl oder -O-Dinitroaryl, insbesondere Phenoxy, 2- oder 4-Nitrophenoxy oder 2,4-Dinitrophenoxy, -$OCH_2CN$ oder den über ein N-Atom gebundenen Rest eines Azols oder Benzazols mit mindestens 2 N-Atomen im quasiaromatischen Fünfring bedeutet.

Die Acylierungsmittel der Formel V stellen somit z. B. Carbonsäurehalogenide, insbesondere Carbonsäurechloride und Carbonsäurebromide, von denen die Carbonsäurechloride bevorzugt sind, Carbonsäuren, Carbonsäureester, Carbonsäureanhydride oder gemischte Carbonsäure-Kohlensäure-Anhydride oder heterocyclische Amide oder Azolide dar.

Bei der Acylierung brauchen die Acylierungsmittel der Formel V, wobei Y die angegebene Bedeutung mit Ausnahme von -OH besitzt, nicht unbedingt in reiner Form eingesetzt zu werden, sondern sie können auch kurz vor der Acylierungsreaktion oder während der Acylierungsreaktion in situ aus den Carbonsäuren der Formel Va

$$R^{10}\text{–CO–OH} \quad \text{(Va)}$$

erzeugt werden. Das heißt, daß als Acylierungsmittel auch die Carbonsäuren der Formel Va verwendet werden können.

Falls als Acylierungsmittel die Carbonsäuren der Formel Va verwendet werden, ist der Zusatz eines Aktivierungsmittels zweckmäßig, das die Aufgabe hat, das Acylierungspotential der Carbonsäure zu erhöhen bzw. zu aktivieren. Als derartige Aktivierungsmittel sind z. B. geeignet:

a) wasserentziehende bzw. wasserbindende Mittel und
b) solche Agenzien, welche die Carbonsäuren der Formel Va in die entsprechenden, als Acylierungsmittel wirkenden Säurehalogenide, Anhydride, Ester, gemischte Carbonsäure-Kohlensäure-Anhydride oder Azolide zu überführen vermögen.

Als geeignete wasserbindende bzw. wasserentziehende Mittel kommen z. B. N,N'-disubstituierte Carbo-diimide der Formel VI

R'–N=C=N–R" (VI)

in Betracht, insbesondere dann, wenn der Rest R' und gegebenenfalls auch der Rest R" ein sekundärer oder tertiärer Alkylrest ist (vgl. Methodicum Chimicum, Verlag G. Thieme Stuttgart, Bd. 6, (1974) S. 682). Geeignete Carbodiimide sind z. B. Di-isopropyl-, Di-cyclohexyl- oder Methyl-tert.-butyl-carbo-diimid. Bei der Durchführung der Acylierungsreaktion werden dann die Verbindung der Formel IV und die Carbonsäure der Formel Va und das Carbo-diimid in einem geeigneten inerten Lösungs- oder Verdünnungsmittel zusammengegeben, wodurch sich das gewünschte Acylierungsprodukt der Formel Ia und aus dem Carbo-diimid der entsprechende disubstituierte Harnstoff bildet.

Beispiele für Agenzien, welche die Carbonsäuren der Formel Va in die entsprechenden Halogenide, Carbonester, Anhydride, gemischte Carbonsäure-Kohlensäure-Anhydride oder Azolide überführen können, sind vor allem Kohlensäurederivate, wie z. B. Phosgen Cl-CO-Cl, Chlorameisensäureester Cl-CO-O-Alkyl, insbesondere mit 1 bis 5 C-Atomen im Alkylrest (vgl. z. B. Tetrahedron Letters *24* (1983) 3365), Kohlensäureester R'"-O-CO-O-R'", wie z. B. N,N'-Disuccinimido-carbonat, Diphthalimido-carbonat, 1,1'-(Carbonyldioxy)-dibenzo-triazol oder Di-2-pyridyl-carbonat (vgl. z. B. Tetrahedron Letters, Vol. 25, No. 43, 4943-4946), gegebenenfalls in Anwesenheit geeigneter Katalysatoren, wie z. B. 4-Dimethylaminopyridin, oder heterocyclische Diamide der Kohlensäure der Formel A-CO-A, worin A ein über ein N-Atom gebundener Rest eines Azols mit mindestens 2 Stickstoffatomen im quasi-aromatischen Fünfring bedeutet. Geeignete derartige heterocyclische Diamide sind z. B. N,N'-Carbonyl-diimidazol, 2,2'-Carbonyl-ditriazol(1,2,3), 1,1'-Carbonyl-ditriazol(1,2,4), N,N'-Carbonyl-dipyrazol, 2,2'-Carbonyl-ditetrazol, N,N'-Carbonylbenzimidazol oder N,N'-Carbonylbenztriazol. Diese Verbindungen werden im allgemeinen vor der eigentlichen Acylierung der Verbindung IV mit der Carbonsäure der Formel Va in einem geeigneten Lösungs- oder Dispergierungsmittel in stöchiometrischen Verhältnissen bei Temperaturen von 0°C bis zum Siedepunkt des Lösungs- oder Verdünnungsmittels, normalerweise bei 10 bis 100°C, vorzugsweise 20 bis 80°C, vereinigt, wobei sich in wenigen Minuten das als eigentliches Acylierungsmittel wirkende Azolid der Formel

$R^{10}$–CO–A

bildet, wobei $R^{10}$ und A die bereits genannten Bedeutungen besitzen. Dieses kann dann sofort im gleichen Topf zur Acylierung des Amins der Formel IV verwendet werden (vgl. z. B. H.A. Staab, M. Lücking u. F.H. Dürr, Chem. Ber. *95*, (1962), 1275, H.A. Staab und W. Rohr "Synthesen mit heterocyclischen Amiden (Azoliden)" in "Neuere Methoden der Präparativen Organischen Chemie", Band V, Verlag Chemie, 1967, S. 53 ff., insbesondere S. 68). Als N,N'-Carbonyldiazol wird häufig das käufliche N,N'-Carbonyl-diimidazol verwendet. Die anderen N,N'-Carbonylazole sind aber aus dem jeweiligen Azol und Phosgen ebenfalls leicht zugänglich.

Als Aktivierungsmittel für die Carbonsäuren der Formel Va können anstelle der Kohlensäurederivate häufig auch die entsprechenden Derivate der Oxalsäure, wie z. B. Oxalylchlorid Cl-CO-CO-Cl (vgl. z. B. GB-PS-2 139 725) oder N,N-Oxalyl-diazole A-CO-CO-A, wobei A die bereits genannte Bedeutung besitz (vgl. z. B. Bull. Chem. Soc. Jap. *57*, 3597-3598 (1984)) eingesetzt werden.

Als Aktivierungsmittel für die Carbonsäuren Va sind jedoch auch andere Verbindungen, wie z. B. Methylethylphosphinsäureanhydrid (vgl. z. B. DE-OS-3 101 427) geeignet.

Die Umsetzung zwischen dem Acylierungsmittel und der Verbindung IV wird zweckmäßigerweise in flüssiger Phase in Anwesenheit eines inerten Lösungs- oder Verdünnungsmittels durchgeführt.

Geeignete Lösungs- oder Verdünnungsmittel sind z. B. solche, die bereits als Lösungs- oder Verdünnungsmittel für die Umsetzung zwischen den Verbindungen II und III genannt worden sind.

Die dabei als Lösungs- oder Verdünnungsmittel angegebenen Alkohole, Glykole und teilweise veretherten Glykole sowie Wasser sind normalerweise nur für die Acylierung mit Carbonsäureestern geeignet, dagegen für die Durchführung der Acylierung mit anderen Acylierungsmitteln wegen der konkurierenden Bildung von Estern, Glykolestern oder Säuren nicht ausreichend inert und daher weniger geeignet.

Das Molverhältnis zwischen der Verbindung der Formel IV und dem Acylierungsmittel der Formel V beträgt 1 : 1. Zweckmäßigerweise wird das Acylierungsmittel in einem geringen molaren Überschuß eingesetzt. Überschüsse von bis zu 30 mol.-% sind in der Regel ausreichend, d.h. das Molverhältnis zwischen der Verbindung der Formel IV und dem Acylierungsmittel der Formel V beträgt normalerweise 1 : (1 bis 1,3), vorzugsweise 1 : (1 bis 1,2). Falls bei der Acylierungsreaktion eine Säure abgespalten wird, ist der Zusatz eines Säurefängers, wie z. B. eines Alkalihydroxids, wie z. B. Natrium-, Kalium- oder Lithiumhydroxids, eines tertiären organischen Amins, wie z. B. Pyridin oder Triethylamin zweckmäßig. Bei der Acylierungsreaktion können auch geeignete Katalysatoren, wie z. B. 4-Dimethylaminopyridin, zugesetzt werden.

Die Umsetzung zwischen dem Acylierungsmittel der Formel V und der Verbindung IV kann prinzipiell bei Temperaturen zwischen -10°C und dem Siedepunkt des verwendeten Lösungs- oder Verdünnungsmittels erfolgen. In vielen Fällen wird die Umsetzung bei 0 bis 50°C, insbesondere bei 10 bis 30°C und vorzugsweise bei Raumtemperatur, durchgeführt.

Zur Herstellung von erfindungsgemäßen Verbindungen der Formel Ia, bei denen $R^{11}$ Wasserstoff bedeutet, kann es zweckmäßig sein, zunächst eine Verbindung der Formel Ia herzustellen, bei der $R^{11}$ einen Rest der Formel $R^{12}$-CO- bedeutet, und diesen Rest anschließend in an sich bekannter Weise, z. B. durch Hydrierung oder Umsetzung mit Säuren oder Basen durch Wasserstoff zu ersetzen.

Sofern die als Acylierungsmittel eingesetzten Carbonsäurederivate ein Asymmetriezentrum besitzen, erhält man durch Umsetzung mit Verbindungen der Formel IV diastereomere Amide. Eingesetzt wurden sowohl enantiomerenreine Carbonsäuren als auch Racemate. Erfindungsgemäße Verbindungen sind die erhaltenen Diastereomerengemische oder diastereomerenreine Amide, die durch Umkristallisation der bei Einsatz von enantiomerenreinen Carbonsäurederivaten erhaltenen Amide herstellbar sind.

Sofern die Verbindungen der Formel I basische Reste enthalten, bilden sie mit Säuren Säureadditionssalze. Zur Bildung derartiger Säureadditionssalze sind anorganische und organische Säuren geeignet. Geeignete Säuren sind beispielsweise: Chlorwasserstoff, Bromwasserstoff, Naphthalindisulfonsäuren, insbesondere 1,5-Napthalindisulfonsäure, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Äpfel-, Sulfamin-, Phenylpropion-, Glucon-, Ascorbin-, Isonicotin-, Methansulfon-, p-Toluolsulfon-, Zitronen- oder Adipin-Säure. Pharmakologisch annehmbare Säureadditionssalze werden bevorzugt. Die Säureadditionssalze können wie üblich durch Vereinigung der Komponenten, zweckmäßigerweise in einem geeigneten Lösungs- oder Verdünnungsmittel, hergestellt werden.

Zweckmäßigerweise wird dabei die Verbindung der Formel I in einem organischen Lösungsmittel gelöst und mit einer Lösung der gewünschten Säure versetzt. So können beispielsweise die Hydrochloride der erfindungsgemäßen Pyridazinone der Formel I dadurch erhalten werden, daß die Verbindung I in Alkohol gelöst wird und die alkoholische Lösung mit einer äquivalenten Menge einer Lösung von Chlorwasserstoff in Diethylether versetzt wird.

Die erfindungsgemäßen 4,5-Dihydro-3(2H)-pyridazinonderivate der Formel I und ihre pharmakologisch annehmbaren Salze zeigen ausgeprägte antithrombotische, thrombozytenaggregationshemmende, antianginöse, kardiotone und blutdrucksenkende Wirkungen. Sie sind den bisher bekannten Verbindungen gleicher Wirkungsrichtung überraschenderweise erheblich überlegen und eignen sich daher vorzüglich am Menschen zur Behandlung und Vorbeugung von Erkrankungen des Herzens und des Kreislaufsystems einschließlich thromboembolischer Erkrankungen. Sie zeigen eine ausgezeichnete Wirksamkeit in verschiedenartigen Tests, wie z. B. Thrombozytenaggregation nach Born, Nature *194*, S. 927, (1961); Arachidonsäureletalität am Kaninchen, Science *193*, S. 1085, (1974): arterielle und venöse Thromboseverhinderung beim Kaninchen, günstiges hämodynamisches Profil am Hund. Die Prüfung in den genannten und einer Reihe weiterer Tests ergibt, daß die erfindungsgemäß herstellbaren Verbindungen bei geringer Toxizität überraschenderweise ein besonders günstiges, bei bekannten Präparaten in dieser Form nicht vorliegendes Wirkungsprofil aufweisen.

Die erfindungsgemäßen 4,5-Dihydro-3(2H)-pyridazinone der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon, neben üblichen pharmazeutisch einwandfreien Träger- und Zusatzstoffen, enthalten.

Die Heilmittel können oral, z. B. in Form von Pillen, Tabletten, Lacktabletten, Dragees, Hart- und Weichgelatinekapseln, Granulaten, Lösungen, Sirupen, Elixieren, Emulsionen oder Suspensionen oder Aerosolmischungen, verabreicht werden. Die Verabreichung kann aber auch rektal, z. B. in Form von Suppositorien, oder parenteral, z. B. in Form von Injektionslösungen, oder perkutan, z. B. in Form von Salben oder Tinkturen, erfolgen.

Die pharmazeutischen Präparate werden in an sich bekannter Weise, z. B. durch Verstrecken der Wirkstoffe mit pharmazeutisch annehmbaren anorganischen und/oder organischen Trägerstoffen und/oder Lösungsmitteln hergestellt. Die pharmazeutischen Präparate können auch zwei oder mehrere Verbindungen der Formel I oder ihrer pharmakologisch annehmbaren Säureadditionssalze enthalten. Normalerweise enthalten die pharmazeutischen Präparate die therapeutisch wirksamen Verbindungen oder das Gemisch therapeutisch wirksamer Verbindungen in einer Konzentration von ca 0,5 bis 90 Gew.-% der Gesamtmischung.

Für die Herstellung von Pillen, Tabletten, Dragees, Hart- und Weichgelatinekapseln oder Granulaten kann man als Trägerstoffe z. B. natürliche Gesteinsmehle, wie z. B. Talkum, Tonerden, Kaoline, Kreide, synthetische Gesteinsmehle, wie z. B. Silikate, Kieselsäure, Zucker, wie z. B. Invert-, Trauben-, Milch-, Malz-, Frucht- oder Rohrzucker, Stärke oder Stärkederivate, wie z. B. Maisstärke, Kartoffelstärke, Gelatine, verwenden. Als Trägerstoffe für Weichgelatinekapseln und Suppositorien sind z. B. Fette, Wachse, Paraffine, wie z. B. Erdölfraktionen, natürliche Öle, wie z. B. Erdnuß- oder Sesamöl, gehärtete Öle, halbfeste und flüssige Polyole etc. geeignet. Als Trägerstoffe für die Herstellung von Lösungen und Sirupen eigenen sich z. B. Wasser, Alkohole, Saccachrose, Invertzucker, Glukose, Polyole etc. Als Trägerstoffe für die Herstellung von Injektionslösungen eignen sich z. B. Wasser, Alkohole, Glykole, Glyzerin, Polyole, pflanzliche Öle etc.

Die pharmazeutische Präparate können in bekannter Weise neben den Wirk- und Trägerstoffen noch Zusatzstoffe, wie z. B. Füllstoffe, Streck-, Spreng-, Binde-, Gleit-, Netz-, Stabilisierungs-, Emulgier-, Dispergier-, Konservierungs-, Süß-, Färbe-, Geschmacks- oder Aromatisierungs-, Dickungs-, Verdünnungsmittel, Zuschlagstoffe, Puffersubstanzen, ferner Lösungsmittel, Hilfslösungsmittel oder Lösungsvermittler oder Mittel zur Erzielung eines Depoteffekts, sowie Salze zur Veränderung des osmotischen Drucks, Überzugsmittel oder Antioxidanten enthalten.

Die tägliche Dosierung kann innerhalb weiter Grenzen, z. B. von 0,001 mg/kg Körpergewicht bis zu 20 mg/kg Körpergewicht variieren und ist in jedem einzelnen Fall den individuellen Gegebenheiten anzupassen.

Je nach Art der Applikation bewegt man sich innerhalb des genannten Dosierungsbereichs, um in bekannter Weise den unterschiedlichen Resorptionsbedingungen Rechnung zu tragen. So wählt man bei intravenöser Applikation eine Dosierung mehr im unteren Teil des angegebenen Dosierungsbereichs. Im allgemeinen wird man bei

intravenöser Applikation tägliche Mengen von etwa 0,001 bis 1 mg/kg, vorzugsweise etwa 0,01 bis 0,5 mg/kg Körpergewicht zur Erzielung wirksamer Ergebnisse verabreichen. Bei oraler Applikation beträgt die tägliche Dosierung in der Regel etwa 0,01 bis 20 mg/kg, vorzugsweise 0,1 bis 10 mg/kg Körpergewicht. Gegebenenfalls kann es erforderlich werden, von den genannten Mengen abzuweichen. Bei der Verabreichung größerer Mengen ist es empfehlenswert, die Tagesdosis in mehrere, z. B. zwei oder drei über den Tagesverlauf verteilte Teilverabreichungen aufzuteilen.

Die pharmazeutischen Präparate können neben den Verbindungen der allgemeinen Formel I auch noch eine oder mehrere andere pharmazeutisch wirksame Substanzen, beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen wie Digoxin, Acetyldigoxin, Metyldigoxin und Lanato-Glykoside; Coronardilatatoren, wie Carbocromen, Dipyridamol, Nifedipin und Perhexilin; antianginöse Verbindungen wie Isosorbiddinitrat, Isosorbidmononitrat, Glyceroltrinitrat, Molsidomin und Verapamil; β-Blocker wie Propranolol, Oxprenolol, Atenolol, Metoprolol und Penbutolol und oogenmetabolische Mittel, wie Pirilinol, enthalten.

Die folgenden Beispiele 1 bis 19 betreffen die Herstellung von Verbindungen der Formel II, die Beispiele 20 bis 75 betreffen die Herstellung von Verbindungen der Formel I, und die Beispiele A bis H betreffen die Herstellung von Zubereitungen der Verbindungen der Formel I.

**Beispiel 1**

**4-((2-Oxo-pyrrolidin-1-yl)-phenyl)-4-oxo-3-methyl-2-butensäure**

66,7 g (0,5 mol) wasserfreies Aluminiumchlorid werden in 150 ml Schwefelkohlenstoff suspendiert. Unter Rühren und Kühlen werden 32,2 g (0,2 mol) N-Phenyl-pyrrolidin-2-on und 22,4 g (0,2 mol) Methylmaleinsäureanhydrid zugegeben. Man erhitzt unter Rückfluß, bis eine dickflüssige Masse entsteht, läßt 40 h bei Raumtemperatur stehen, dekantiert das Lösungsmittel ab, zersetzt vorsichtig mit Eiswasser und konz. wäßriger Salzsäure und extrahiert mit Methylenchlorid oder Essigsäureethylester. Die organische Phase wird mit verdünnter Natronlauge extrahiert, die Wasserphase klarfiltriert, mit Essigsäure angesäuert, extrahiert und eingeengt. Das erhaltene Öl wird direkt weiter umgesetzt.

Ausbeute: 37,6 g (69 % der Theorie).

**Beispiel 2**

**4-((2-Oxo-pyrrolidin-1-yl)-phenyl)-4-oxo-3-methyl-buttersäure**

27,3 g (0,1 mol) der in Beispiel 1 erhaltenen Verbindung werden in 200 ml Wasser und 15 ml Essigsäure gelöst. Nach Zugabe von 15 g (0,23 mol) Zinkstaub wird 30 min unter Rückfluß erhitzt und abfiltriert. Die Wasserphase wird alkalisch gestellt, extrahiert, angesäuert und das erhaltene Öl abgetrennt.

Ausbeute: 12,7 g (46 % der Theorie).

**Beispiel 3**

**4-(4-(2-(Imidazol-1-yl)-ethoxy)-phenyl)-4-oxo-3-methyl-buttersäure-methylester**

40 g (0,3 mol) wasserfreies Aluminiumchlorid werden in 100 ml wasserfreiem 1,2-Dichlorethan suspendiert und auf 10°C abgekühlt. Unter Kühlen werden 16,5 g (0,1 mol) 4-Methoxy-carbonyl-2-methyl-buttersäurechlorid in 50 ml 1,2-Dichlorethan und 18,8 g (0,1 mol) 1-(2-Phenoxy)-ethyl-imidazol in 50 ml 1,2-Dichlorethan zugegeben. Man rührt 16 h bei Raumtemperatur, hydrolisiert mit Eiswasser und wäßriger konz. Salzsäure. Nach Zugabe von 27 %-iger Natronlauge wird bei pH 13 schnell extrahiert, getrocknet und eingeengt.

Ausbeute: 28,3 g (90 % der Theorie).

**Beispiel 4**

**4-(4-(2-Oxo-imidazolidin-1-yl)-phenyl)-4-oxo-3-methylbuttersäure**

20 g (0,15 mol) wasserfreies Aluminiumchlorid werden in 100 ml Nitrobenzol gelöst. Bei 5°C werden 8,1 g (0,05 mol) 2-Oxo-1-phenyl-imidazolidin und 8,2 g (0,05 mol) 3-Methoxycarbonyl-2-methyl-buttersäurechlorid in 100 ml Nitrobenzol innerhalb 1 h zugetropft. Nach 20 h Reaktionszeit bei 50°C wird hydrolysiert, mit Essigsäureethylester extrahiert, getrocknet und eingeengt.

Der erhaltene Ester wird mit 380 ml 0,7 %-iger wäßriger Natronlauge versetzt und bei Raumtemperatur 20 h

gerührt. Nach Extraktion mit Methylenchlorid wird klarfiltriert und mit Salzsäure auf pH 1 angesäuert, abgetrennt und umkristallisiert.

Ausbeute: 4,1 g (30 % der Theorie), Fp.: 166 - 167°C.

Analog den vorstehenden Beispielen können alle benötigten Ketocarbonsäurederivate der Formel II hergestellt werden.

**Als Beispiele seien genannt:**

5. 4-(4-(3-oxo-pyrazolidin-1-yl)-phenyl)-4-oxo-3-methyl-buttersäure und -methyl-ester

6. 4-(3-Methyl-1-phenyl-5-oxo-pyrazol-4-yl)-4-oxo-3-methyl-buttersäure und -methyl-ester

7. 4-(4-((2-Oxo-oxazolidin-5-yl)-methoxy)-phenyl)-4-oxo3-methyl-buttersäure und -methyl-ester

8. 4-(3-(2,4-Dioxo-imidazolidin-1-yl)-4-methoxy-phenyl)4-oxo-3-methyl-buttersäure und -methyl-ester

9. 4-(4-(2-(2-Oxo-pyrrolidin-1-yl)-ethyl)-amino-carbonyl-aminophenyl)-4-oxo-3-methyl-buttersäure und -methylester

10. 4-(2-Methoxyethoxy-phenyl)-4-oxo-3-methyl-buttersäure und -methyl-ester, -ethyl-ester und n-propyl-ester

11. 4-(5-Amino-1,3-dimethyl-pyrazol-4-yl)-4-oxo-3-methylbuttersäure und -methyl-ester

12. 4-(4-(3-Pyridyl-methoxy) -phenyl)-4-oxo-3-methyl-buttersäure und -methyl-ester

13. 4-(4-(Aminocarbonyl-methoxy)-phenyl)-4-oxo-3-methylbuttersäure und -methyl-ester

14. 4-(4-Methylthio-phenyl)-4-oxo-3-methyl-buttersäure und -methyl-ester, -ethyl-ester und n-propyl-ester

15. 4-(4-(5-Methyl-1,3,4-oxadiazol-2-yl)-methoxy-phenyl)-4-oxo-3-methyl-buttersäure und -methyl-ester

16. 4-(4-(2-Hydroxy-4-methyl-pyrid-6-yl)-methoxy-phenyl)4-oxo-3-methyl-buttersäure und -methyl-ester

17. 4-(4-(2-(2-Methoxy-carbonyl-pyrrolidin-1-yl)-ethoxy)phenyl)-4-oxo-3-methyl-buttersäure und -methyl-ester

18. 4-(4-(2,5-Dioxo-pyrrolidin-1-yl)-phenyl)-4-oxo-3methyl-buttersäure und -methyl-ester

19. 4-(3-Indolyl)-4-oxo-3-methyl-buttersäure und -methyl-ester, -ethyl-ester und n-propyl-ester

**Beispiel 20**

**6-(4-(2-(Imidazol-1-yl)-ethoxy)-phenyl)-5-methyl-4,5-dihydro-3(2H)-pyridazinon**

12,7 g (0,04 mol) der in Beispiel 3 hergestellten Verbindung und 3 ml (0,06 mol) Hydrazinhydrat werden in 100 ml Methanol 30 min bei Raumtemperatur und anschließend 2 h unter Rückfluß erhitzt. Nach dem Einengen wird mit wäßriger Natriumcarbonatlösung und Methylenchlorid versetzt, die organische Phase abgetrennt, eingeengt und aus Isopropanol umkristallisiert.

Ausbeute: 2,9 g (24 % der Theorie), Fp.: 151 - 153°C
Elementaranalyse: $C_{16}H_{18}N_4O_2$ (298.35)

| | | | | |
|---|---|---|---|---|
| ber.: | C 64,4 | H 6,1 | N 18,8 | O 10,7 |
| gef.: | C 63,9 | H 5,9 | N 18,9 | O 11,0 |

**Beispiel 21**

**6-(4-(2-Oxo-pyrrolidin-1-yl)-phenyl)-5-methyl-4,5-dihydro-3(2H)-pyridazinon**

6,9 g (0,025 mol) der in Beispiel 2 hergestellten Verbindung werden mit 1,5 ml (0,03 mol) Hydrazinhydrat in 30 ml Ethanol 1 h unter Rückfluß erhitzt. Das ausgefallene Produkt wird abgesaugt und aus Isopropanol umkristallisiert.

Ausbeute: 5,5 g (81 % der Theorie), Fp.: 204 - 205°C
Elementaranalyse: $C_{15}H_{17}N_3O_2$ (271.32)

| | | | | |
|---|---|---|---|---|
| ber.: | C 66,4 | H 6,3 | N 15,5 | O 11,8 |
| gef.: | C 66,2 | H 5,9 | N 15,4 | O 12,1 |

**Beispiel 22**

**6-(4-(2-Oxo-imidazolidin-1-yl)-phenyl)-5-methyl-4,5-dihydro-3(2H)-pyridazinon**

4 g (0,014 mol) der in Beispiel 4 hergestellten Verbindung werden mit 1 ml (0,021 mol) Hydrazinhydrat in 30 ml Ethanol und 20 ml Dimethylformamid 2 h bei 80°C gerührt. Nach dem Abkühlen wird abgesaugt und aus Dimethylformamid umkristallisiert.

Ausbeute: 0,6 g (16 % der Theorie), Fp.: 322 - 325°C
Elementaranalyse: $C_{14}H_{16}N_4O_2$ (272.31)

| | | | | |
|---|---|---|---|---|
| ber.: | C 61,8 | H 5,9 | N 20,6 | O 11,8 |
| gef.: | C 61,4 | H 6,1 | N 20,7 | O 11,7 |

**Beispiel 23**

**6-(4-(2-Methoxy-ethoxy)-phenyl)-4,5-dihydro-3(2H)-pyridazinon**

6 g (0,024 mol) 4-(4-(2-Methoxy-ethoxy)-phenyl)-4-oxo-buttersäure und 1,2 g (0,024 mol) Hydrazinhydrat werden in 90 ml Ethanol 1 h bei 50°C gerührt. Nach dem Abkühlen wird abgesaugt, mit Ethanol gewaschen und getrocknet.

Ausbeute: 5,2 g (88 % der Theorie), Fp.: 146 - 148°C
Elementaranalyse: $C_{13}H_{16}N_2O_3$ (248.28)

| | | | | |
|---|---|---|---|---|
| ber.: | C 62,9 | H 6,5 | H 11,3 | O 19,3 |
| gef.: | C 63,4 | H 6,4 | N 11,3 | O 19,0 |

**Beispiel 24**

**6-(4-(2-Oxo-pyrrolidin-1-yl)-phenyl)-4,5-dihydro-3(2H)-pyridazinon**

10,4 g (0,04 mol) 4-(4-(2-Oxo-pyrrolidin-1-yl)-phenyl)-4-Oxo-buttersäure und 2 g (0,04 mol) Hydrazinhydrat werden in 100 ml Ethanol 1 h unter Rückfluß gerührt, abgekühlt und abgesaugt. Der ausgefallene Feststoff wird mit Natriumhydrogencarbonatlösung verrührt, abgesaugt, gewaschen und getrocknet.

Ausbeute: 9 g (87 % der Theorie), Fp.: 272 - 274°C
Elementaranalyse: $C_{14}H_{15}N_3O_2$

| | | | | |
|---|---|---|---|---|
| ber.: | C 65,4 | H 5,9 | N 16,3 | O 12,4 |
| gef.: | C 64,7 | H 6,2 | N 16,2 | O 12,7 |

**Beispiel 25**

**6-(4-(2-Oxo-imidazolidin-1-yl)-phenyl)-4,5-dihydro-3(2H)-pyridazinon**

7,9 g (0,03 mol) 4-(4-(2-Oxo-imidazolidin-1-yl)-phenyl)-4-oxo-buttersäure und 1,5 g (0,03 mol) Hydrazinhydrat werden in 100 ml Ethanol 2 h bei 50°C und 3 h unter Rückfluß erhitzt. Nach dem Abkühlen wird abgesaugt, gewaschen, getrocknet und aus DMF umkristallisiert.

Ausbeute: 1,8 g (23 % der Theorie), Fp.: > 300°C
Elementaranalyse: $C_{13}H_{14}N_4O_2$ (258.28)

| | | | | |
|---|---|---|---|---|
| ber.: | C 60,5 | H 5,5 | N 21,7 | O 12,4 |
| gef.: | C 60,3 | H 5,1 | N 21,7 | O 12,6 |

**Beispiel 26**

**6-( 4-( 2-( 2-Oxo-pyrrolidin-1-yl )ethyl )-amino-carbonyl-amino-phenyl )-5-methyl-4,5-dihydro-3( 2H )-pyridazinon**

7,2 g (0,02 mol) 4-(4-(2-(2-Oxo-pyrrolidin-1-yl)-ethyl)amino-carbonyl-amino-phenyl)-4-oxo-3-methyl-buttersäure und 1 ml (0,02 mol) Hydrazinhydrat werden in 80 ml Ethanol und 10 ml Dimethylformamid 2 h unter Rückfluß erhitzt. Das beim Abkühlen ausgefallene Rohprodukt (6,3 g) wird abgesaugt und aus 90 %-igem Ethanol umkristallisiert.

Ausbeute: 3,8 g (53 % der Theorie), Fp.: 280 - 282°C
Elementaranalyse: $C_{18}H_{23}N_5O_3$ (357.42)

| | | | | |
|---|---|---|---|---|
| ber.: | C 60,5 | H 6,5 | N 19,6 | O 13,4 |
| gef.: | C 60,5 | H 6,5 | N 19,8 | O 13,5 |

**Beispiel 27**

**6-( 4-Methoxy-3-( 2,4-dioxo-imidazolidin-1-yl-phenyl )-5-methyl-4,5-dihydro-3(2H)-pyridazinon**

8,6 g (0,027 mol) 4-(3-(2,4-Dioxo-imidazolidin-1-yl-)-4-methoxy-phenyl)-4-oxo-3-methyl-buttersäure und 1,5 ml (0,031 mol) Hydrazinhydrat werden in 100 ml Ethanol und 20 ml DMF 4 h unter Rückfluß erhitzt. Man rührt noch 20 h bei Raumtemperatur, filtriert ab und kristallisiert aus Ethanol um.

Ausbeute: 6,4 g (75 % der Theorie), Fp.: 292 - 294°C
Elementaranalyse: $C_{15}H_{16}N_4O_4$ (316.32)

| | | | | |
|---|---|---|---|---|
| ber.: | C 57,0 | H 5,1 | N 17,7 | O 20,2 |
| gef.: | C 56,1 | H 5,1 | N 17,2 | O 21,0 |

**Beispiel 28**

**6-(4-(2-Oxo-oxazolidin-5-yl)-methoxy-phenyl)-5-methyl-4,5-dihydro-3(2H)-pyridazinon**

5,9 g (0,019 mol) 4-(4-(2-Oxo-oxazolidin-5-yl)-methoxy-phenyl)-4-oxo-3-methyl-buttersäure und 1,5 ml (0,031 mol) Hydrazinhydrat werden in 30 ml Methanol 1 h unter Rückfluß erhitzt.

Nach Zugabe von Petrolether kristallisiert das Produkt aus. Das Rohprodukt wird aus 96 %-igem Isopropanol umkristallisiert.

Ausbeute: 1,35 g (23 % der Theorie), Fp.: 199 - 201°C
Elementaranalyse: $C_{15}H_{17}N_3O_4$ (303.32)

| | | | | |
|---|---|---|---|---|
| ber.: | C 59,4 | H 5,6 | N 13,9 | O 21,1 |
| gef.: | C 59,8 | H 5,3 | N 13,4 | O 21,6 |

**Beispiel 29**

**6-(4-(5-Hydroxy-3-methyl-1-phenyl-pyrazolyl)-5-methyl-4,5-dihydro-3(2H)-pyridazinon**

4,5 g (0,016 mol) 4-4-(5-Hydroxy-3-methyl-1-phenyl-pyrazolyl)-4-oxo-3-methyl-buttersäure und 1,5 ml (0,031 mol) Hydrazinhydrat werden in 30 ml Ethanol 1 h unter Rückfluß erhitzt. Nach dem Einengen wird mit Isopropanol aufgekocht, abfiltriert und das auskristallisierte Produkt abgetrennt.

Ausbeute: 2,5 g (56 % der Theorie), Fp.: 215 - 217°C
Elementaranalyse: $C_{15}H_{16}N_4O_2$ (284.32)

| | | | | |
|---|---|---|---|---|
| ber.: | C 63,4 | H 5,7 | N 19,7 | O 11,3 |
| gef.: | C 62,3 | H 5,7 | N 18,6 | O 11,9 |

**Beispiel 30**

**6-( 4-( 5-Methyl-1,3,4-oxadiazol-2-yl )-methoxy-phenyl )-5-methyl-4,5-dihydro-3(2H)-pyridazinon**

10,1 g (0,033 mol) 4-(4-(5-Methyl-1,3,4-Oxadiazol-2-yl)-methoxy-phenyl)-4-oxo-3-methyl-buttersäure und 2,1 ml (0,043 mol) Hydrazinhydrat werden in 100 ml Ethanol 1 h bei 50°C gerührt. Nach Zugabe einer Mischung von Diethyl-ether/Ligroin bis zur Trübung kristallisiert das Produkt aus. Nach dem Absaugen wird aus 500 ml Isopropanol umkristallisiert.

Ausbeute: 3,3 g (33 % der Theorie), Fp.: 142 - 144°C
Elementaranalyse: $C_{15}H_{16}N_4O_3$ (300.32)

| | | | | |
|---|---|---|---|---|
| ber.: | C 60,0 | H 5,4 | N 18,7 | O 16,0 |
| gef.: | C 60,6 | H 5,8 | H 18,8 | O 15,2 |

**Beispiel 31**

**6-(3-Indolyl)-5-methyl-4,5-dihydro-3(2H)-pyridazinon**

7,0 g (0,03 mol) 4-(3-(Indolyl)-4-oxo-3-methylbuttersäure und 1,7 ml (0,035 mol) Hydrazinhydrat werden in 60 ml Ethanol 2 h unter Rückfluß erhitzt. Nach dem Einengen wird über eine Kieselgelsäule mit Methylenchlorid : Methanol = 9 : 1 als Laufmittel chromatographiert und aus Isopropanol umkristallisiert.

Ausbeute: 1,5 g (22 % der Theorie), Fp.: 257 - 259°C
Elementaranalyse: $C_{13}H_{13}N_3O$ (227.27)

| | | | | |
|---|---|---|---|---|
| ber.: | C 68,7 | H 5,8 | N 18,5 | O 7,0 |
| gef.: | C 68,3 | H 5,7 | H 18,1 | O 7,3 |

**Beispiel 32**

**6-(4-(Hydroxycarbonyl-methoxy)-phenyl-5-methyl-4,5-dihydro-3-(2H)-pyridazinon**

8,0 g (0,03 mol) (4-(4-Hydroxycarbonyl-methoxy)-phenyl)-4-oxo-3-methylbuttersäure und 2,5 ml (0,052 mol) Hydrazinhydrat werden in 100 ml n-Propanol 1 h unter Rückfluß erhitzt.

Nach dem Einengen wird mit Natriumhydrogencarbonatlösung versetzt, mit Methylenchlorid extrahiert, die Wasserphase angesäuert und das ausgefallene Produkt aus Isopropanol umkristallisiert.

Ausbeute: 1,7 g (22 % der Theorie), Fp.: 224 - 226°C
Elementaranalyse: $C_{13}H_{14}N_2O_4$ (262.27)

| | | | | |
|---|---|---|---|---|
| ber.: | C 59,5 | H 5,4 | N 10,7 | O 24,4 |
| gef.: | C 59,1 | H 5,2 | N 10,8 | O 24,6 |

**Beispiel 33**

**6-(4-Methylthio-phenyl)-5-methyl-4,5-dihydro-3(2H)-pyridazinon**

2,3 g (0,01 mol) 4-(4-Methylthio-phenyl)-4-oxo-3-methyl-buttersäure und 1 ml Hydrazinhydrat werden in 20 ml Isopropanol 1 h unter Rückfluß erhitzt. Nach dem Abkühlen wird abgesaugt und getrocknet.

Ausbeute: 1,8 g (80 % der Theorie), Fp.: 199 - 201°C
Elementaranalyse: $C_{12}H_{14}N_2OS$ (234.32)

| | | | | |
|---|---|---|---|---|
| ber.: | C 61,5 | H 6,0 | N 12,0 | O 6,8 |
| gef.: | C 61,8 | H 6,0 | N 12,1 | O 6,7 |

**Beispiel 34**

**6-(4-(3-Pyridylmethyl)-phenyl)-5-methyl-4,5-dihydro-3(2H)-pyridazinon-hydrochlorid**

7,1 g (0,025 mol) 4-(4-(3-Pyridylmethyl)-phenyl)-4-oxo-3-methyl-buttersäure und 1,5 ml (0,03 mol) Hydrazinhydrat werden in 30 ml Ethylenglykolmonomethylether 1 h bei 100°C gerührt. Nach Zugabe von ethanolischer Salzsäure wird abgesaugt und aus 96 % Ethanol umkristallisiert.

Ausbeute: 4,4 g (56 % der Theorie), Fp.: 225 - 226°C
Elementaranalyse: $C_{17}H_{18}ClN_3O$ (315.81)

| | | | | | |
|---|---|---|---|---|---|
| ber.: | C 64,7 | H 5,7 | Cl 11,2 | N 13,3 | O 5,1 |
| gef.: | C 65,1 | H 5,7 | Cl 11,8 | N 13,5 | O 5,2 |

Analog den vorgenannten Beispielen können z. B. die folgenden 4,5-Dihydro-3(2H)-pyridazinone der Formel I hergestellt werden:

**Beispiel 35**

**6-(4-(2,5-Dioxo-pyrrolidin-1-yl)-phenyl)-5-methyl-4,5-dihydro-3(2H)-pyridazinon**

Lösungsmittel: Ethanol
Reaktionstemperatur: 75°C
Ausbeute: 62 % der Theorie
Schmelzpunkt: 225 bis 227°C

**Beispiel 36**

**6-(4-(5-Amino-1,3-dimethyl-pyrazolyl)-5-methyl-4,5-dihydro-3(2H)-pyridazinon**

Lösungsmittel: n-Butanol
Reaktionstemperatur: 100°C
Ausbeute: 66 % der Theorie

**Beispiel 37**

**6-(4-(3-Pyridyl)-methoxy-phenyl)-5-methyl-4,5-dihydro-3(2H)-pyridazinon**

Lösungsmittel: n-Pentanol
Reaktionstemperatur: 70°C
Ausbeute: 43 % der Theorie
Schmelzpunkt: Hydrochlorid 217 bis 218°C

**Beispiel 38**

**6-(4-(Aminocarbonyl)-methoxy-phenyl)-5-methyl-4,5-dihydro-3(2H)-pyridazinon**

Lösungsmittel: Dimethylformamid
Reaktionstemperatur: 100°C
Ausbeute: 63 % der Theorie
Schmelzpunkt: 226 bis 227°C

**Beispiel 39**

**6-( 4-( ( 2-Methoxy-ethyl )-amino-carbonyl )-methoxy-phenyl )-5-methyl-4,5-dihydro-3( 2H )-pyridazinon**

Lösungsmittel: Dimethylsulfoxid
Reaktionstemperatur: 90°C
Ausbeute: 51 % der Theorie
Schmelzpunkt: 182 bis 183°C

**Beispiel 40**

**6-(4-(2,4-Dioxo-imidazolidin-1-yl-)-phenyl-5-methyl-4,5-dihydro-3(2H)-pyridazinon**

Lösungsmittel: Ethylenglykol
Reaktionstemperatur: 160°C
Ausbeute: 34 % der Theorie

**Beispiel 41**

**6-( 4-( 4-Methyl-2-hydroxy-pyridyl-6-yl )-methoxyphenyl )-5-methyl-4,5-dihydro-3( 2H )-pyridazinon**

Lösungsmittel: 1,4-Dioxan
Reaktionstemperatur: 70°C
Ausbeute: 47 % der Theorie
Schmelzpunkt: 127 bis 128°C

**Beispiel 42**

**6-( 4-( 2-( 2-Methoxycarbonyl-pyrrolidin-1-yl )-ethoxy )-phenyl )-5-methyl-4,5-dihydro-3( 2H )-pyridazinon**

Lösungsmittel: Dibutylether
Reaktionstemperatur: 140°C
Ausbeute: 82 % der Theorie

**Beispiel 43**

**6-(4-(2-Oxo-imidazolidin-1-yl)-phenyl)-2,5-dimethyl-3(2H)-pyridazinon**

Lösungsmittel: Pyridin
Reaktionstemperatur: 60°C
Ausbeute: 78 % der Theorie

**Beispiel 44**

**6-(4-(3-Oxo-pyrazolidin-1-yl)-phenyl)-5-methyl-4,5-dihydro-3(2H)-pyridazinon**

Lösungsmittel: Cyclohexanol
Reaktionstemperatur: 65°C
Ausbeute: 25 % der Theorie
Schmelzpunkt: 154 bis 155°C

**Beispiel 45**

**6-(4-Methyl-sulfonyl-phenyl)-5-methyl-4,5-dihydro-3(2H)-pyridazinon**

Lösungsmittel: 1,2-Dimethoxyethan
Reaktionstemperatur: 50°C
Ausbeute: 54 % der Theorie

**Beispiel 46**

**6-(4-Methyl-sulfinyl-phenyl)-5-methyl-4,5-dihydro-3(2H)-pyridazinon**

Lösungsmittel: Xylol
Reaktionstemperatur: 90°C
Ausbeute: 87 % der Theorie

**Beispiel 47**

**6-(2-Pyrrolyl)-5-methyl-4,5-dihydro-3(2H)-pyridazinon**

Lösungsmittel: Tetrahydrofurand
Reaktionstemperatur: 60°C
Ausbeute: 38 % der Theorie
Schmelzpunkt: 196 bis 198°C

**Beispiel 48**

**6-(3-Indolyl)-2-isopropyl-5-methyl-4,5-dihydro-3(2H)-pyridazinon**

Lösungsmittel: Acetonitril
Reaktionstemperatur: 80°C
Ausbeute: 73 % der Theorie

**Beispiel 49**

**6-(4-(4-Methyl-2-oxo-pyran-6-yl)-methoxy-phenyl)-5-methyl-4,5-dihydro-3(2H)-pyridazinon**

Lösungsmittel: Chlorbenzol
Reaktionstemperatur: 70°C
Ausbeute: 47 % der Theorie

**Beispiel 50**

**6-(4-(2-Oxo-oxazolidin-3-yl)-phenyl)-5-methyl-4,5-dihydro-3(2H)-pyridazinon**

Lösungsmittel: Chloroform
Reaktionstemperatur: 60°C
Ausbeute: 31 % der Theorie

**Beispiel 51**

**6-(L-4-Thiazolidin-4-yl-carbonyl-amino-phenyl)-5-methyl-4,5-dihydro-3(2H)-pyridazinon**

1,9 g (0,014 mol) L-Thiazolidin-4-carbonsäure, 2,9 g (0,014 mol) 6-(4-Amino-phenyl)-5-methyl-4,5-dihydro-3(2H)-pyridazinon und 8 ml Triethylamin werden in 10 ml Dimethylformamid gelöst. Nach Abkühlen auf 0 bis 5°C werden langsam 5 ml (0,03 mol) Methylethylphosphinsäureanhydrid zugetropft. Man rührt 6 h bei Raumtemperatur, hydrolysiert mit wäßriger Natriumhydrogencarbonatlösung und kristallisiert das ausgefallene Produkt aus Isopropanol um.

Ausbeute: 1,1 g (25 % der Theorie), Fp.: 214 - 216°C
Elementaranalyse: $C_{15}H_{10}N_4O_2S$ (318.40)

| | | | | |
|---|---|---|---|---|
| ber.: | C 56,6 | H 5,7 | N 13,6 | O 10,0 |
| gef.: | C 55,9 | H 5,5 | N 13,6 | O 10,2 |

**Beispiel 52**

**6-(4-(3-tert.-Butyloxycarbonyl-L-thiazolidin-4-yl-carbonyl-amino)-phenyl)-5-methyl-4,5-dihydro-3(2H)-pyridazinon**

9,3 g (0,04 mol) 3-tert.-Butyloxycarbonyl-L-thiazolidin-4-carbonsäure und 7,0 g (0,04 mol) 1,1'-Carbonyl-ditriazol-(1,2,4) werden in 15 ml N-Methylpyrrolidinon 15 min bei 60°C gerührt. Nach Zugabe von 7,1 g (0,035 mol) 6-(4-Aminophenyl)-5-methyl-4,5-dihydro-3(2H)-pyridazinon wird 6 h bei Raumtemperatur gerührt, mit Wasser versetzt, extrahiert, mit Methanol kristallisiert und aus Methanol/Essigsäureethylester umkristallisiert.

Ausbeute: 6,5 g (39 % der Theorie), Fp.: 200 bis 201°C
Elementaranalyse $C_{20}H_{26}N_4O_4S$ (418.52)

| | | | | | |
|---|---|---|---|---|---|
| ber.: | C 57,4 | H 6,3 | N 13,4 | O 15,3 | S 7,7 |
| gef.: | C 57,3 | H 6,0 | N 13,1 | O 15,6 | S 7,6 |

**Beispiel 53**

**6-(4-(3-Benzyloxycarbonyl-L-thiazolidin-4-yl-carbonyl-amino)-phenyl)-5-methyl-4,5-dihydro-3(2H)-pyridazinon**

8,0 g (0,03 mol) 3-Benzyloxycarbonyl-L-thiazolidin-4-carbonsäure und 4,9 g (0,03 mol) N,N'-Carbonyl-diimidazol werden in 10 ml Dimethylformamid 10 min bei 60°C gerührt. Nach Zugabe von 5,1 g (0,025 mol) 6-(4-Aminophenyl)-5-methyl-4,5-dihydro-3(2H)-pyridazinon wird 18 h bei Raumtemperatur gerührt, eingeengt, mit wäßriger Natriumhydrogencarbonatlösung versetzt, mit Methylenchlorid extrahiert und über eine Kieselgelsäule (Laufmittel: Methylenchlorid : Methanol = 95 : 5) chromatographiert.

Ausbeute: 7,7 g (43 % der Theorie),
Elementaranalyse: $C_{23}H_{24}N_4O_4S$ (452.54)

| | | | | |
|---|---|---|---|---|
| ber.: | C 61,0 | H 5,3 | N 12,4 | O 14,1 |
| gef.: | C 60,5 | H 5,3 | N 12,5 | O 14,1 |

Das N,N'-Carbonyl-diimidazol kann mit ähnlich gutem Erfolg durch eine äquivalente Menge 2,2'-Carbonyl-di-triazol(1,2,3), 1,1'-Carbonyl-di-triazol(1,2,4), 1,1'-Carbonyl-di-pyrazol, 2,2'-Carbonyl-di-tetrazol, N,N'-Carbonyl-di-benzimidazol oder N,N'-Carbonyl-di-benztriazol ersetzt werden.

**Beispiel 54**

**6-(4-(5-Oxa-perhydro-(1,4)-thiazepin-3-yl-carbonyl-amino)-phenyl)-5-methyl-4,5-dihydro-3(2H)-pyridazinon**

5,3 g (0,03 mal) 5-Oxo-perhydro-(1,4)-thiazepin-3-carbonsäure und 6,1 g (0,03 mol) N,N'-Carbonyl-diimidazol werden in 15 ml Dimethylformamid 15 min bei 50°C gerührt. Nach Zugabe von 4,9 g (0,03 mol) 6-(4-Amino-phenyl)-5-methyl-4,5-dihydro-3(2H)-pyridazinon wird 12 h bei Raumtemperatur gerührt, abgesaugt und getrocknet.

Ausbeute: 6,0 g (55 % der Theorie), Fp.: 272 bis 274°C
Elementaranalyse: $C_{17}H_{20}N_4O_3S$ (360.44)

| | | | | | |
|---|---|---|---|---|---|
| ber.: | C 56,7 | H 5,6 | N 15,5 | O 13,3 | S 8,9 |
| gef.: | C 56,7 | H 5,4 | N 15,6 | O 13,6 | S 8,8 |

**Beispiel 55**

**6-(4-(3-tert.-Butyloxycarbonyl-thiazolidin-2-yl-carbonyl-amino)-phenyl)-5-methyl-4,5-dihydro-3(2H)-pyridazinon**

7,5 g (0,032 mol) 3-tert.-Butyloxycarbonyl-thiazolidin-2-carbonsäure und 5,2 g (0,032 mol) N,N'-Carbonyl-diimidazol werden in 50 ml Tetrahydrofuran 15 min unter Rückfluß erhitzt. Nach Zugabe von 6,1 g (0,03 mol) 6-(4-Amino-phenyl)-5-methyl-4,5-dihydro-3(2H)-pyridazinon wird 18 h bei Raumtemperatur gerührt, eingeengt und mit Essigsäureethylester kristallisiert.

Ausbeute: 4,8 g (38 % der Theorie), Fp.: 225 bis 226°C
Elementaranalyse: $C_{20}H_{26}N_4O_4S$ (418.52)

| | | | | | |
|---|---|---|---|---|---|
| ber.: | C 57,4 | H 6,3 | N 13,4 | O 15,3 | S 7,7 |
| gef.: | C 57,4 | H 5,6 | N 13,7 | O 15,2 | S 7,7 |

**Beispiel 56**

**6-(4-(4-Chlorphenoxyacetylamine)-phenyl)-5-methyl-4,5-dihydro-3(2H)-pyridazinon**

Zu 5,1 g (0,025 mol) 6-(4-Amino-phenyl)-5-methyl-4,5-dihydro-3(2H)-pyridazinon in 20 ml Pyridin werden 0,1 g 4-Dimethylaminopyridin und 5,1 g (0,025 mol) 4-Chlorphenoxyacetylchlorid zugegeben. Man rührt 5 h bei Raumtemperatur, engt ein, versetzt mit Wasser und extrahiert mit Methylenchlorid.

Ausbeute: 6,0 g (65 % der Theorie), Fp.: 226 bis 227°C
Elementaranalyse: $C_{19}H_{18}ClN_3O_3$ (371.82)

| | | | | | |
|---|---|---|---|---|---|
| ber.: | C 61,4 | H 4,9 | Cl 9,5 | N 11,3 | O 12,9 |
| gef.: | C 60,7 | H 4,8 | Cl 9,0 | N 11,4 | O 13,2 |

**Beispiel 57**

**6-( 4-( Thiazolidin-2-yl-carbonyl-amino-phenyl )-5-methyl-4,5-dihydro-3(2H)-pyridazinon-hydrochlorid**

4,2 g (0,01 mol) 6-(4-(3-tert.-Butyloxycarbonyl-thiazolidin-2-yl-carbonylamino)-phenyl)-5-methyl-4,5-dihydro-3(2H)-pyridazinon werden bei 0°C mit 15 ml eishalter Trifluoressigsäure versetzt. Man neutralisiert im Eisbad mit Natronlauge, stellt auf pH 4 mit Kaliumhydrogensulfatlösung und extrahiert mit Methylenchlorid. Nach Zugabe von methanolischer Salzsäure wird das ausgefallene Produkt abgesaugt und aus Isopropanol/Essigsäureethylester umkristallisiert.

Ausbeute: 1,9 g (54 % der Theorie), Fp.: 233 bis 234°C
Elementaranalyse: $C_{15}H_{19}ClN_4O_2S$ (354.86)

| | | | | |
|---|---|---|---|---|
| ber.: | C 50,8 | H 5,4 | N 15,8 | O 9,0 |
| gef.: | C 50,5 | H 5,6 | N 15,4 | O 9,1 |

**Beispiel 58**

**6-( 4-( S-( 4-Pyridyl )-thioacetylamino )-phenyl )-5-methyl-4,5-dihydro-3(2H)-pyridazinon-hydrochlorid**

3,4 g (0,02 mol) S-4-Pyridylmercapto-essigsäure und 3,3 g (0,02 mol) N,N'-Carbonyldiimidazol werden in 20 ml Dimethylformamid (DMF) 15 min bei 60°C gerührt. Nach Zugabe von 4,1 g (0,02 mol) 6-(4-Amino-phenyl)-5-methyl-4,5-dihydro-3(2H)-pyridazinon wird 5 h bei Raumtemperatur gerührt, mit Wasser versetzt und abgesaugt.

Das Produkt wird in 96 %-igem Ethanol gelöst, filtriert, mit ethanolischer Salzsäure versetzt und abgesaugt.

Ausbeute: 5,8 g (74 % der Theorie), Fp.: 278 bis 280°C
Elementaranalyse: $C_{18}H_{19}ClN_4O_2S$ (390.89)

| | | | | | | |
|---|---|---|---|---|---|---|
| ber.: | C 55,3 | H 4,9 | Cl 9,1 | N 14,3 | O 8,2 | S 8,2 |
| gef.: | C 55,3 | H 4,9 | Cl 9,2 | N 14,0 | O 8,1 | S 8,3 |

Analog den vorstehenden Beispielen 52 bis 58 lassen sich die in den nachfolgenden Beispielen 57 bis 69 angegebenen Verbindungen herstellen:

**Beispiel 59**

**6-( 4-( Perhydro-1,4-thiazin-3-yl )-carbonyl-amino )-phenyl-5-methyl-4,5-dihydro-3(2H)-pyridazinon**

Lösungsmittel: Dioxan
Reaktionstemperatur: 70°C
Ausbeute: 53 % der Theorie

**Beispiel 60**

**6-( 4-( Perhydro-4-formyl-1,4-thiazin-3-yl )-carbonyl-amino )phenyl-5-methyl-4,5-dihydro-3(2H)-pyridazinon**

Lösungsmittel: Tetrahydrofuran
Reaktionstemperatur: 60°C
Ausbeute: 68 % der Theorie

**Beispiel 61**

**6-( 4-( Perhydro-3-oxo-1,4-thiazin-5-yl )-carbonyl-amino )-phenyl-5-methyl-4,5-dihydro-3(2H)-pyridazinon**

Lösungsmittel: Dimethylformamid
Reaktionstemperatur: 100°C
Ausbeute: 71 % der Theorie

**Beispiel 62**

**6-( 4-( 3-Formyl-thiazolidin-4-yl )-carbonyl-amino )-phenyl-5-methyl-4,5-dihydro-3(2H)-pyridazinon**

Lösungsmittel: Ethylenglykoldimethylether
Reaktionstemperatur: 85°C
Ausbeute: 42 % der Theorie

**Beispiel 63**

**6-( 4-( 3-Acetyl-thiazolidin-4-yl )-carbonyl-amino )-phenyl-5-methyl-4,5-dihydro-3(2H)-pyridazinon**

Lösungsmittel: Diethylenglykoldimethylether
Reaktionstemperatur: 100°C
Ausbeute: 49 % der Theorie

**Beispiel 64**

**6-( 4-( Perhydro-1,3-thiazin-4-yl )-carbonyl-amino )-phenyl-5-methyl-4,5-dihydro-3(2H)-pyridazinon**

Lösungsmittel: Dimethylsulfoxid
Reaktionstemperatur: 110°C
Ausbeute: 27 % der Theorie

**Beispiel 65**

**6-( 4-( Perhydro-3-tert.-butyloxycarbonyl-1,3-thiazin-4-yl )-carbonyl-amino )-phenyl-5-methyl-4,5-dihydro-3(2H)-pyridazinon**

Lösungsmittel: Methylenchlorid
Reaktionstemperatur: 40°C
Ausbeute: 79 % der Theorie

**Beispiel 66**

**6-( 4-( Perhydro-3-formyl-1,3-thiazin-4-yl )-carbonyl-amino )-phenyl-5-methyl-4,5-dihydro-3(2H)-pyridazinon**

Lösungsmittel: Hexamethylphosphorsäuretriamid
Reaktionstemperatur: 90°C
Ausbeute: 65 % der Theorie

**Beispiel 67**

**6-( 4-( 2-Oxo-thiazolidin-4-yl )-carbonyl-amino )-phenyl-5methyl-4,5-dihydro-3(2H)-pyridazinon**

Lösungsmittel: Methanol
Reaktionstemperatur: 25°C
Ausbeute: 25 % der Theorie

**Beispiel 68**

**6-(4-(Thiazolidin-2-yl)-carbonyl-amino)-phenyl-5-methyl-4,5-dihydro-3(2H)-pyridazinon**

Lösungsmittel: Chlorbenzol
Reaktionstemperatur: 120°C
Ausbeute: 32 % der Theorie

**Beispiel 69**

**6-( 4-( 1-Oxido-3-oxo-perhydro-1,4-thiazin-5-yl )-carbonyl-amino )-phenyl-5-methyl-4,5-dihydro-3(2H)-pyridazinon**

Lösungsmittel: Ethanol
Reaktionstemperatur: 80°C
Ausbeute: 39 % der Theorie

**Beispiel 70**

**6-( 4-( 1-Oxido-3-oxo-perhydro-1,4-thiazin-5-yl )-carbonylamino )-phenyl-5-methyl-4,5-dihydro-3(2H)-pyridazinon**

Lösungsmittel: Dimethylformamid
Reaktionstemperatur: 90°C
Ausbeute: 84 % der Theorie

**Beispiel 71**

**6-( 4-( 1-Oxido-5-oxo-perhydro-1,4-thiazepin-3-yl )-carbonyl-amino )-phenyl-5-methyl-4,5-dihydro-3(2H)-pyridazinon**

Lösungsmittel: Acetonitril
Reaktionstemperatur: 81°C
Ausbeute: 61 % der Theorie

**Beispiel 72**

**6-(4-(3-Pyridyl-oxy)-acetylamino)-phenyl-5-methyl-4,5-dihydro-3(2H)-pyridazinon**

Lösungsmittel: Pyridin
Reaktionstemperatur: 115°C
Ausbeute: 75 % der Theorie

**Beispiel 73**

**6-(4-(3-Pyridyl-methoxy)-acetylamino)-phenyl-5-methyl-4,5-dihydro-3(2H)-pyridazinon**

Lösungsmittel: Ethylenglykolmonomethylether
Reaktionstemperatur: 100°C
Ausbeute: 42 % der Theorie

**Beispiel 74**

**6-(4-(4-Pyridyl-sulfinyl)-acetylamino)-phenyl-5-methyl-4,5-dihydro-3(2H)-pyridazinon**

Lösungsmittel: Dioxan
Reaktionstemperatur: 70°C
Ausbeute: 67 % der Theorie

**Beispiel 75**

**6-(4-(4-Pyridyl-sulfonyl)-acetylamino)-phenyl-5-methyl-4,5-dihydro-3(2H)-pyridazinon**

Lösungsmittel: N-Methylpyrrolidon
Reaktionstemperatur: 100°C
Ausbeute: 48 % der Theorie

Die folgenden Beispiele veranschaulichen die Zusammensetzung von Zubereitungen der erfindungsgemäßen 4,5-Dihydro-3(2H)-pyridazinone.

**Beispiel A**

| Tabletten | pro Tablette |
|---|---|
| Wirkstoff (feingemahlen) | 50 mg |
| Milchzucker | 150 mg |
| Maisstärke, weiß | 230 mg |
| Polyvinylpyrrolidon | 15 mg |
| Magnesiumstearat | 5 mg |
| | 450 mg |

**Beispiel B**

| Injektionslösung | |
|---|---|
| Wirkstoff | 4 mg |
| Natriumchlorid | 0,7 mg |
| Wasser zu Injektionszwecken ad | 1 ml |

**Beispiel C**

| Rektale Arzneiform | |
|---|---|
| Wirkstoff | 20 mg |
| Suppositoriumsgrundmasse ad | 2 g |

**Beispiel D**

| Emulsionen | |
|---|---|
| Wirkstoff | 60 mg |
| Glycerin, rein | 0,2 - 2,0 g |
| Polyethylenstearat | q.s. |
| Neutralöl | q.s. |
| Geschmackskorrigens | q.s. |
| Wasser entsalzt ad | 100 ml |

**Beispiel E**

| Wirkstofflösungen | |
|---|---|
| Wirkstoff | 8 mg |
| Polyethylenglykol | 1,5 mg |
| Glycofurol ad | 4 ml |
| Wasser zu Injektionszwecken | 6 ml |

**Beispiel F**

| Tabletten | pro Tablette |
|---|---|
| Wirkstoff | 20 mg |
| Lactose | 60 mg |
| Maisstärke | 30 mg |
| lösliche Stärke | 5 mg |
| Magnesiumstearat | 5 mg |
| | 120 mg |

**Beispiel G**

| Dragees | |
|---|---|
| Wirkstoff | 6 mg |
| Propanolol | 40 mg |
| Milchzucker | 90 mg |
| Maisstärke | 90 mg |
| sec. Calciumphosphat | 34 mg |
| | 260 mg |

**Beispiel H**

| Kapseln | |
|---|---|
| Wirkstoff | 5 mg |
| Prazosin | 5 mg |
| Maisstärke | 185 mg |
| | 195 mg |

Die erfindungsgemäßen Tetrahydropyridazinonderivate zeigen bereits in geringer Dosierung eine therapeutisch besonders wertvolle Kombination von antithrombotischer, cardiotoner und antianginöser Wirkung bei geringer Blutdrucksenkung.

Die folgenden Tabellen 1 bis 5 zeigen die in verschiedenen in vivo- und in vitro Tests erhaltenen Wirkungsdaten erfindungsgemäßer Verbindungen.

Die in der Tabelle 1 angegebenen Werte des arteriellen Thromboseschutzes wurden an der Ratte nach der Methode von Meng und Seuter (Naunyn-Schmiedeberg's Arch. Pharmacol., *301*, 115 (1977)), die des venösen Thromboseschutzes am Kaninchen nach der Methode von Harbauer ("Versuche zur Entwicklung eines standardisierten venösen Thrombosemodells am Kaninchen", 17. Angiologisches Symposium in Kitzbühel, (1982)) ermittelt.

Die in der Tabelle 2 angegebenen Werte der Beeinflussung der Arachidonsäurewirkung wurden am narkotisierten Meerschweinchen nach der Methode von Lefort und Vargaftig (Br. J. Pharmac. *63*, 35 (1978)) bestimmt.

Die in der Tabelle 3 angegebenen Werte der Inhibierung der Thrombozytenaggregation wurden in vitro nach der Methode von Born (J. Physiol. *162*, 67 P (1962)) unter Einsatz von Arachidonsäure, Thrombin, Collagen, PAF (Platelet Activating Factor Acether) und von Adenosindiphosphat (ADP) als Aggregationsmittel ermittelt.

Die direkte positiv inotrope Wirkung der Substanzen wurde am isolierten, elektrostimulierten Meerscheinchenvorhof getestet. Dazu wurden die Meerschweinchen durch Genickschlag getötet, das Herz rasch entfernt, der linke Vorhof freipräpariert und in einem thermostatisierten 4-fach-Organbad (Fa. HSE Typ "Schuler") bei einer Vorspannung von 1 g montiert. Die Kontraktionskraft der elektrisch stimulierten linken Vorhöfe (Frequenz 120/min, Spannung ca 15 V, Impulsbreite 1 msec) wurde isometrisch mit Statham UC-2-Kraftaufnehmern erfaßt, und das verstärkte Signal auf einem 4-kanaligen Schreiber (Linear Corder Mark VII, Fa. Watanabe) kontinuierlich aufgezeichnet.

Nach einer Stabilisierung von ca 60 min erfolgte die kumulative Prüfsubstanz-Gabe in Abständen von 30 min. Pro Substanz wurden 4 Vorhöfe verwendet. Jeder erhielt nur eine Substanz.

Die Auswertung erfolgte jeweils kurz vor Gabe der nächsthöheren Dosis. Aus den Einzelwerten wurden $\bar{X}$ und $S\bar{X}$ berechnet und die Substanzwirkung als δ % Änderung auf den Ausgangswert vor der 1. Dosis bezogen. Die erhaltenen Werte sind in der Tabelle 4 angegeben.

Zum Nachweis der antianginösen Wirkung der erfindungsgemäßen Verbindungen wurden Untersuchungen an Bastardhunden beiderlei Geschlechts in Pentobarbital-Narkose (30 bis 40 mg/kg i.v.) oder in Urethan-Chloralose-Narkose (3 ml/kg Urethan-Chloralose-Gemisch i.v. = 20 mg/kg Chloralose und 250 mg/kg Urethan) durchgeführt. Die Beatmung der Tiere erfolgte mit einem Bird-Mark-7-Respirator. Der endexpiratorische Kohlensäuregehalt (gemessen mit einem Ultrarotabsorptionsschreiber) betrug zwischen 4,5 und 5 Vol.-%. Während des gesamten Versuchs erhielten die Tiere mit Pentobarbital-Narkose eine Dauerinfusion von Pentobarbital i.v. = 4 mg (in 6 ml)/kg/h, um eine konstante Narkosetiefe zu gewährleisten. Die Tiere mit Urethan-Chloralose erhielten keine Dauerinfusion. Die Infusion wurde durch die Vena cephalica gegeben. Nach der Präparation des Versuchstieres wurde ca. 1 h gewartet, bis sich alle haemodynamischen Parameter eingestellt hatten (steady state). Danach wurde mit dem eigentlichen Versuch begonnen.

Der systolische und diastolische Blutdruck wurden peripher in der Arteria femoralis über einen Statham-Druckaufnehmer gemessen. Ein über die Arteria carotis in den linken ventrikel geschobener Millar-Tip-Katheter lieferte das Signal für den linksventrikulären enddiastolischen Druck (= LVEDP) und die Herzfrequenz (= HF). Außerdem wurde die Druckanstiegsgeschwindigkeit in der linken Herzkammer als Maß der Kontraktilität des Herzens bestimmt.

Die erhaltenen Ergebnisse sind in der Tabelle 5 angegeben.

Bei der in den Tabellen aufgeführten Vergleichsubstanz handelt es sich um Amrinon.

**Tabelle 1**

Beeinflussung der experimentellen Thrombose in vivo.

| Verbindung des Beispiels Nr. | Dosis mg/kg i.p. | Thromboseschutz (%) arteriell (Ratte) | Thromboseschutz (%) venös (Kaninchen) |
|---|---|---|---|
| 58 | 10 | 70 | 100 |
| | 1 | 40 | 25 |
| 35 | 10 | 50 | 100 |
| | 3 | | 88 |
| 21 | 10 | 25 | 67 |
| 22 | 10 | – | 50 |
| 20 | 10 | 36 | 100 |
| | 1 | | 33 |
| 28 | 10 | – | 43 |
| 30 | 10 | 50 | 100 |
| | 3 | – | 75 |
| 31 | 10 | – | 29 |
| 51 | 10 | 60 | 100 |
| | 1 | 50 | 100 |
| | 0,3 | – | 50 |
| 54 | 10 | – | 33 |
| 55 | 10 | 47 | 57 |
| 56 | 10 | 22 | 50 |
| 52 | 10 | 70 | 100 |
| | 3 | 40 | 75 |
| Vergleichssubstanz | 10 | – | 29 |

**Tabelle 2**

Beeinflussung der Arachidonsäurewirkungen (Arachidonsäure 500 µg/kg i.v.) in vivo beim narkotisierten Meerschweinchen (Änderung in %)

| Verbindung des Beispiels Nr. | Dosis mg/kg i.v. | $TXA_2$-Effekt Broncho-spasmus | $TXA_2$-Effekt Thrombo-zytopenie | $PGI_2$-Effekt Blutdruck-senkung |
|---|---|---|---|---|
| 58 | 0,01 | – | –32 | +35 |
| | 0,03 | –19 | –90 | +32 |
| 35 | 1 | – 7 | –44 | – |
| | 3 | –11 | –43 | – |
| 21 | 0,1 | –58 | –75 | +35 |
| | 0,3 | –67 | –73 | – |
| 20 | 1 | – | – | +70 |
| 28 | 1 | –81 | –89 | –26 |
| | 10 | –87 | –58 | +67 |
| 30 | 1 | –36 | –75 | – |
| 51 | 0,1 | –67 | –87 | –36 |
| | 0,3 | –82 | –100 | –26 |
| 54 | 1 | – | –72 | –38 |
| 55 | 0,1 | –54 | –49 | +37 |
| | 0,3 | –74 | –71 | +49 |
| 52 | 0,01 | – | –47 | – |
| | 0,03 | – | –69 | – |
| Vergleichssubstanze | 1 | –47 | –42 | – |

**Tabelle 3**

Hemmung der Thrombozytenaggregation in vitro (in $IC_{50}$ µMol)

| Verbindung des Beispiels Nr. | A | B | C | D | E |
|---|---|---|---|---|---|
| 58 | 0,06 | 30 | 0,015 | 2 | 20 |
| 35 | 0,6 | 15 | – | 3 | 20 |
| 21 | 0,1 | 2 | 0,2 | 0,35 | 1 |
| 22 | 0,6 | 20 | – | – | 10 |
| 20 | 0,35 | 7 | 3,5 | 2 | 45 |
| 28 | 0,3 | 5,5 | 1 | 3 | 4 |
| 30 | 0,075 | 6 | 0,07 | 1,5 | 8 |
| 31 | 0,2 | 6,5 | – | 1,5 | 8,5 |
| 51 | 0,7 | 50 | – | 1 | 20 |
| 54 | 0,4 | 2 | – | – | 1 |
| 55 | 0,04 | 2 | 0,03 | 0,55 | 0,2 |
| 56 | 0,55 | 10 | – | – | 5 |
| 52 | 0,008 | 0,3 | 0,02 | 0,03 | 0,04 |
| Vergleichssubstanz | 9 | > 100 | – | 55 | 100 |

In der vorstehenden Tabelle bedeuten:

A = durch 0,36 mmol Arachidonsäure induziert
B = durch 10 µmol ADP (Adenosin-diphosphat) induziert
C = durch 0,2 - 0,4 N.I.H-(National Institute of Health-)Einheiten/ml Thrombin induziert
D = durch 0,1 µmol PAF (Platelet Activating Factor Acether) induziert
E = durch 5 µg/ml Collagen induziert

**Tabelle 4**

Bestimmung der kardiotonen Wirkung am isolierten linken Meerschweinchenvorhof

| Verbindung des Beispiels Nr. | Dosis (mol) | Kraftänderung in % |
|---|---|---|
| 21 | $10^{-3}$ | +46 |
| 22 | $10^{-4}$ | –23 |
| 20 | $10^{-3}$ | +61 |
| 28 | $10^{-6}$ | +13 |
| 30 | $10^{-3}$ | +30 |
| 31 | $10^{-3}$ | +63 |
| 51 | $10^{-3}$ | –30 |
| 54 | $10^{-3}$ | –37 |
| 55 | $3 \cdot 10^{-4}$ | +89 |
| 56 | $10^{-6}$ | +14 |
| 52 | $10^{-5}$ | +60 |
| Vergleichssubstanz | $10^{-4}$ | +34 |

**Tabelle 5**

Herz-Kreislauf-Screening am normotonen, narkotisierten Hund

| Verbindung des Beispiels Nr. | Dosis (mg/kg) | Bd (mmHg) syst. | diast. | LVEDP (mm Hg) | HF (S/min) | dP/dt (mm Hg/s) |
|---|---|---|---|---|---|---|
| 58 | 1,0 i.v. | −75 | −45 | −2 | +14 | + 300 |
| 35 | 1,0 i.d. | −30 | −20 | −9 | +35 | +2000 |
| 21 | 1,0 i.d. | −60 | −45 | −4 | +40 | +1250 |
| 22 | 5,0 i.v. | −60 | −40 | −3 | +35 | +3300 |
| 20 | 1,0 i.d. | −10 | −15 | −3 | +38 | + 900 |
| 28 | 1,0 i.v. | −30 | −15 | −5 | +16 | +1500 |
| 30 | 1,0 i.v. | −50 | −25 | −3 | +19 | +2400 |
| 31 | 3,0 i.d. | −25 | −15 | −2 | +15 | +1000 |
| 51 | 5,0 i.d. | −50 | −35 | −4 | + 9 | +1100 |
| 54 | 1,0 i.v. | −40 | −40 | −9 | +33 | +2900 |
| 55 | 1,0 i.v. | −60 | −45 | −3 | +40 | + 600 |
| 56 | 1,0 i.v. | −40 | −30 | −3 | +18 | +1200 |
| Vergleichssubstanz | 1,0 i.v. | −10 | −15 | −1 | +15 | + 750 |
| | 3,0 i.v. | −30 | −30 | −2 | +15 | +1200 |

In der vorstehenden Tabelle bedeuten:

ΔBd = Änderung des systolischen und diastolischen Blutdrucks
ΔLVEDP = Änderung des linksventrikulären enddiastolischen Drucks
ΔHF = Änderung der Herzfrequenz in S/min = Schläge/min
ΔdP/dt = Änderung der Druckanstiegsgeschwindigkeit in der linken Herzkammer als Maß für die Kontraktilität des Herzens

**Patentansprüche** für die Vertragsstaaten: BE, CH, DE, FR, GB, IT, LI, LU, NL, SE

1. Substituierte 4,5-Dihydro-3(2H)-pyridazinone der Formel I

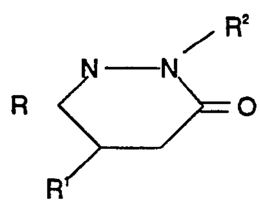

(I)

worin

R einen Rest der Formel

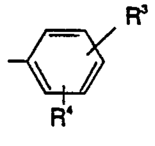

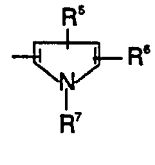

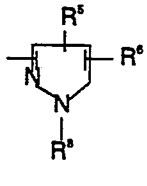

oder

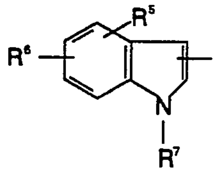

$R^1$, $R^2$ unabhängig voneinander Wasserstoff oder $(C_1-C_4)$Alkyl,

$R^3$ $(C_1-C_4)$Alkoxy-$(C_1-C_4)$alkoxy, Amino-carbonyl-$(C_1-C_4)$alkoxy, $(C_1-C_4)$Alkoxy-$(C_1-C_4)$alkyl-amino-carbonyl-$(C_1-C_4)$alkoxy, Thiazolidinyl-carbonyl-amino, Mono- $(C_1-C_4)$alkylamino-carbonyl-$(C_1-C_4)$alkoxy, $(C_1-C_4)$Alkoxy-carbonyl-$(C_1-C_4)$alkoxy, Hydroxy-carbonyl-$(C_1-C_4)$alkoxy, $(C_1-C_4)$Alkyl-thio, $(C_1-C_4)$Alkylsulfinyl, $(C_1-C_4)$Alkyl-sulfonyl, (2-Oxo-pyrrolidinyl)-$(C_1-C_4)$alkyl-amino-carbonyl-amino, (2-Oxo-piperidinyl)-$(C_1-C_4)$-alkyl-amino-carbonyl-amino, 2-Oxo-pyrrolidinyl, 2-Oxo-piperidinyl, 2,5-Dioxo-piperidinyl, 2,5-Dioxo-pyrrolidinyl, 2-Oxo-imidazolidinyl, 2-Oxo-hexahydro-pyrimidinyl, 2,4-Dioxo-imidazolidinyl, 2,4-Dioxo-hexahydro-pyrimidinyl, 2-Oxo-1,3-oxazolidinyl, 3-Oxo-pyrazolidinyl, (2-($R^9$-carbonyl)-pyrrolidinyl-$(C_1-C_4)$alkoxy, $(C_1-C_4)$Alkyl und $(C_1-C_4)$-Alkoxy substituiert durch Pyridyl, Imidazolyl, Oxadiazolyl, Oxo-pyranyl, 2-Hydroxy-pyridinyl, Pyrrolinyl oder Oxo-oxazolidinyl, wobei das Oxo-pyranyl, Oxo-oxazolidinyl und Oxadiazolyl seinerseits durch $(C_1-C_4)$Alkyl oder $(C_1-C_4)$Alkoxy-carbonyl substituiert sein kann, oder einen Rest der Formel

$R^{10}$–CO–NH–,

$R^4$ Wasserstoff, $(C_1-C_4)$Alkyl, $(C_1-C_4)$-Alkoxy, Hydroxy, $(C_1-C_5)$Alkanoyloxy oder Halogen,

$R^5$, $R^6$, $R^7$ unabhängig voneinander Wasserstoff, $(C_1-C_4)$Alkyl, $(C_1-C_4)$Alkoxy, Hydroxy, Halogen, Amino, Mono-$(C_1-C_4)$alkylamino oder Di-$(C_1-C_4)$alkylamino,

$R^8$ Wasserstoff, $(C_1-C_4)$Alkyl oder Phenyl,

$R^9$ Hydroxy, $(C_1-C_4)$Alkoxy, Amino, Mono-$(C_1-C_4)$alkylamino oder Di-$(C_1-C_4)$alkylamino,

$R^{10}$ p-Chlorphenoxymethyl, 3-Pyridyl-oxy-methyl, 3-Pyridyl-methoxy-methyl, 4-Pyridyl-thiomethyl, 4-Pyridyl-sulfinyl-methyl, 4-Pyridyl-sulfonyl-methyl, 2-Oxo-thiazolidin-4-yl, 3-Oxo-perhydro-1,4-thiazin-5-yl, 1-Oxido-3-oxo-perhydro-1,4-thiazin-5-yl, 5-Oxo-perhydro-1,4-thiazepin-3-yl, 1-Oxido-5-oxo-perhydro-1,4-thiazepin-3-yl, 1,1-Dioxido-5-oxo-perhydro-1,4-thiazepin-3-yl, oder einen Rest der Formel

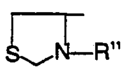 ; 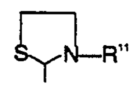

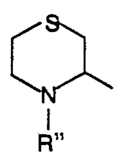 ; 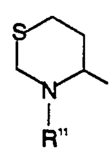

$R^{11}$ Wasserstoff oder einen Rest der Formel

$R^{12}$–CO–,

$R^{12}$ Wasserstoff, Alkyl mit 1 bis 5 C-Atomen, Alkoxy mit 1 bis 5 C-Atomen, Benzyl oder Benzyloxy bedeuten, und ihre pharmakologisch annehmbaren Säureadditionssalze.

2. Verbindungen der Formel I nach Anspruch 1, dadurch gekennzeichnet, daß

$R^1$, $R^2$ unabhängig voneinander Wasserstoff und/oder $(C_1-C_4)$Alkyl,

$R^3$ $(C_1-C_4)$Alkoxy-$(C_1-C_4)$Alkoxy, Amino-carbonyl-$(C_1-C_4)$alkoxy, Mono-$(C_1-C_4)$alkylamino-carbonyl-$(C_1-C_4)$alkoxy, $(C_1-C_4)$Alkoxy-carbonyl-$(C_1-C_4)$alkoxy, $(C_1-C_4)$Alkoxy-$(C_1-C_4)$alkyl-amino-carbonyl-$(C_1-C_4)$alkoxy, Thiazolidinyl-carbonyl-amino, Hydroxy-carbonyl$(C_1-C_4)$alkoxy, $(C_1-C_4)$Alkyl-thio, $(C_1-C_4)$Alkyl-sulfinyl, $(C_1-C_4)$Alkylsulfonyl, (2-Oxo-pyrrolidin-1-yl)-$(C_1-C_4)$alkyl-amino-carbonyl-amino, (2-Oxo-piperidin1-yl)-$(C_1-C_4)$alkyl-amino-carbonyl-amino, 2-Oxo-pyrrolidin-1-yl, 2-Oxo-piperidin-1-yl, 2,5-Dioxo-piperidin-1-yl, 2,5-Dioxo-pyrrolidin-1-yl, 2-Oxo-imidazolin-1-yl, 2-Oxo-hexahydro-pyrimidin-1-yl, 2,4-Dioxo-imidazolin-1-yl, 2,4-Dioxo-hexahydro-pyrimidin-1-yl, 2-Oxo-1,3-oxazolidin-5-yl, 2-Oxo-1,3-oxazolidin-1-yl, 3-Oxo-pyrazolidin-1-yl, (2-($R^9$-carbonyl)-pyrrolidin-1-yl)-alkoxy, $(C_1-C_4)$Alkyl und $(C_1-C_4)$Alkoxy substituiert durch Pyridyl, Imidazolyl, Oxadiazolyl, Oxo-pyranyl, 2-Hydroxy-pyridinyl, Pyrrolinyl oder Oxo-oxazolidinyl, wobei das Oxo-pyranyl, Oxo-oxazolidinyl und Oxadiazolyl seinerseits durch $(C_1-C_4)$Alkyl oder $(C_1-C_4)$Alkoxycarbonyl substituiert sein kann,

$R^4$ Wasserstoff, $(C_1-C_4)$Alkyl, $(C_1-C_4)$Alkoxy, Hydroxy, Alkanoyloxy mit 1 bis 5 C-Atomen, Chlor, Brom,

$R^5$, $R^6$, $R^7$ unabhängig voneinander Wasserstoff, - $(C_1-C_4)$Alkyl, $(C_1-C_4)$Alkoxy, Hydroxy, Chlor, Brom, Amino, Mono-$(C_1-C_4)$alkylamino oder Di-$(C_1-C_4)$alkylamino,

$R^8$ Wasserstoff, $(C_1-C_4)$Alkyl oder Phenyl,

$R^9$ Hydroxy, $(C_1-C_4)$Alkyl, Amino, Mono-$(C_1-C_4)$alkylamino oder Di-$(C_1-C_4)$alkylamino bedeuten.

3. Verbindungen der Formel I nach Anspruch 1 und/oder 2, dadurch gekennzeichnet, daß $R^1$ und $R^2$ unabhängig voneinander Wasserstoff oder Methyl bedeuten oder daß $R^1$ für Methyl und $R^2$ für Wasserstoff steht.

4. Verbindungen der Formel I nach einem oder mehreren der Ansprüche 1 bis 3, dadurch gekennzeichnet, daß R Pyrrolyl, Indolyl, 5-Amino-1,3-dimethyl-pyrazol-4-yl, 5-Hydroxy-3-methyl-1-phenyl-pyrazol-4-yl, bedeutet, oder daß R ein durch $R^3$ und $R^4$ substituierter Phenylrest bedeutet, insbesondere daß R ein durch $R^3$ in 4-Stellung substituierter Phenylrest bedeutet.

5. Verbindungen der Formel I nach einem oder mehreren der Ansprüche 1 bis 4, dadurch gekennzeichnet, daß $R^4$ $(C_1C_4)$Alkyl, $(C_1$-$C_4)$Alkoxy, insbesondere daß $R^4$ Wasserstoff bedeutet.

6. Verbindungen der Formel I nach einem oder mehreren der Ansprüche 1 bis 5, dadurch gekennzeichnet, daß $R^3$ 2-Methoxy-ethoxy, 3-Pyridyl-methoxy, Amino-carbonyl-methoxy, Hydroxy-carbonyl-methoxy, Methylthio, (2-Methoxy-ethyl)-amino-carbonyl-methoxy, 3-Pyridyl-methyl, 5-Methyl-1,3,4-oxadiazol-2-yl, 2-Hydroxy-4-methyl-pyrid-6-yl-methoxy, 2-(Imidazol-1-yl)-ethoxy, (2-Oxo-oxazolidin-5-yl)-methoxy, 2-(2-Oxo-pyrrolidin-1-yl)-ethyl-amino-carbonyl-amino, 2-(Methoxycarbonyl-pyrrolidin-1-yl)-ethoxy, 2-Oxo-pyrrolidin-1-yl, 2,5-Dioxopyrrolidin-1-yl, 2-Oxo-imidazolidin-1-yl, 3-Oxo-pyrazolidin-1-yl, 2,4-Dioxoimidazolidin-1-yl, 2-Oxo-oxazolidin-3-yl, Thiazolidin-4-yl-carbonyl-amino bedeutet.

7. Verbindungen der Formel I nach einem oder mehreren der Ansprüche 1, 3 und/oder 4, dadurch gekennzeichnet, daß $R^1$ Methyl, $R^2$ Wasserstoff, R einen Rest der Formel

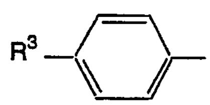

und

$R^3$ einen Rest der Formel

$R^{10}$–CO–NH–

$R^{10}$ p-Chlorphenoxymethyl, 3-Pyridyl-oxy-methyl, 3-Pyridyl-methoxy-methyl, 4-Pyridyl-thiomethyl, 4-Pyridyl-sulfinyl-methyl, 4-Pyridyl-sulfonyl-methyl, 2-Oxo-thiazolidin-4-yl, 3-Oxo-perhydro-1,4-thiazin-5-yl, 1-Oxido-3-oxo-perhydro-1,4-thiazin-5-yl, 5Oxo-perhydro-1,4-thiazepin-3-yl, 1-Oxido-5-oxo-perhydro- 1,4-thiazepin-3-yl, 1,1-Dioxido-5-oxo-perhydro-1,4-thiazepin-3-yl, oder einen Rest der Formel

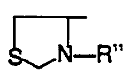 ; 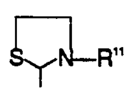

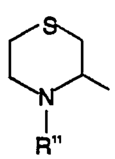 ; 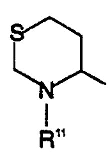

bedeuten und worin $R^{11}$ die im Anspruch 1 angegebene Bedeutung besitzt, und ihre pharmakologisch annehmbaren Säureadditionssalze.

8. 4,5-Dihydro-3(2H)-pyridazinone nach Anspruch 7, dadurch gekennzeichnet, daß $R^{11}$ Wasserstoff, Formyl, Acetyl, tert.-Butoxy-carbonyl oder Benzyloxycarbonyl bedeutet.

9. Verfahren zur Herstellung der substituierten 4,5-Dihydro-3(2H)-pyridazinone der Formel I eines der mehrerer der Ansprüche 1 bis 6, dadurch gekennzeichnet, daß eine Carbonsäure oder ein Carbonsäurederivat der Formel II

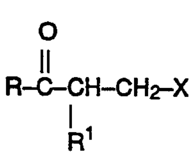

(II)

worin R und $R^1$ die in den Ansprüchen 1 bis 6 angegebene Bedeutung besitzt und X = -COOH, -COCl, -CO-O-CO-$R^{13}$, -CO-O$R^{13}$ oder -CN und $R^{13}$ ein organischer Rest bedeuten, mit einem Hydrazin der Formel III

$H_2N{-}NHR^2$ (III)

worin $R^2$ die in den Ansprüchen 1 bis 6 angegebene Bedeutung besitzt, umgesetzt und die erhaltene Verbindung gegebenenfalls in ein Säureadditionssalz überführt wird, oder daß gegebenenfalls eine Verbindung der allgemeinen Formel I mit $R^2$ = H in eine Verbindung der allgemeinen Formel I mit $R^2$ = Alkyl durch Alkylierung mit einem den Alkylrest $R^2$ einführenden Alkylierungsmittel überführt wird, und dann gegebenenfalls in ein Säureadditionssalz überführt wird.

10. Verfahren zur Herstellung der in den Ansprüchen 7 oder 8 angegebenen 4,5-Dihydro-3(2H)-pyridazinone, dadurch gekennzeichnet, daß 6-(4-Aminophenyl)-4,5-dihydro-3(2H)-pyridazinon der Formel IV

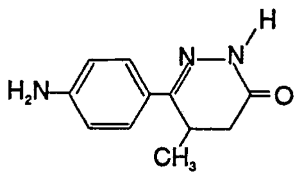

(IV)

mit einem Acylierungsmittel, das den Acylrest $R^{10}$-CO-einführt, umgesetzt wird und die entstehende Verbindung gegebenenfalls in ein Säureadditionssalz überführt wird, wobei als Acylierungsmittel vorzugsweise eine Verbindung der Formel V

$R^{10}{-}CO{-}Y$ (V)

worin $R^{10}$ die in Anspruch 7 angegebene Bedeutung besitzt und Y = Halogen, insbesondere -Cl oder -Br, -OH, Alkyl, -O-Alkyl, insbesondere mit 1 bis 5 C-Atomen, -O-CO-$R^{10}$ oder -O-CO-O-Alkyl, insbesondere mit 1 bis 5 C-Atomen im Alkylrest, -O-Aryl, -O-Nitroaryl oder -O-Dinitroaryl, insbesondere Phenoxy, 2- oder 4-Nitrophenoxy oder 2,4-Dinitrophenoxy, -$OCH_2CN$ oder den über ein N-Atom gebundenen Rest eines Azols oder Benzazols mit mindestens 2 N-Atomen im quasi-aromatischen Fünfring bedeutet, eingesetzt wird, wobei die Carbonsäure der Formel Va

$R^{10}{-}CO{-}OH$ (Va)

vorzugsweise zusammen mit einem Aktivierungsmittel als Acylierungsmittel eingesetzt wird.

11. Verwendung der substituierten 4,5-Dihydro-3-(2H)-pyridazinone der Formel I eines oder mehrerer der Ansprüche 1 bis 8 oder ihrer pharmakologisch annehmbaren Säureadditionssalze für die Herstellung eines Heilmittels zur Bekämpfung und Vorbeugung von Erkrankungen des Herzens und des Kreislaufsystems, einschließlich thromboembolischer Erkrankungen.

12. Pharmazeutische Zubereitung, enthaltend eine wirksame Dosis eines substituierten 4,5-Dihydro-3-(2H)-pyridazinons der Formel I eines oder mehrerer der Ansprüche 1 bis 8 oder eines pharmakologisch annehmbaren Säureadditionssalzes davon neben pharmakologisch zulässigen Träger- und gegebenenfalls Zusatzstoffen.

**Patentansprüche** für den Vertragsstaat: AT

1. Verfahren zur Herstellung substituierter 4,5-Dihydro-3(2H)-pyridazinone der Formel I

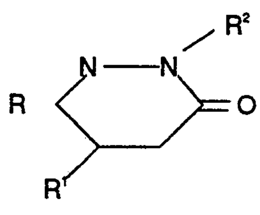

(I)

worin

R einen Rest der Formel

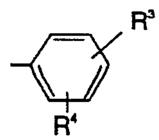 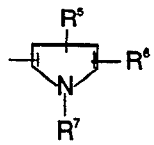

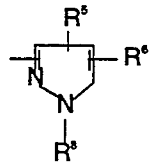 oder 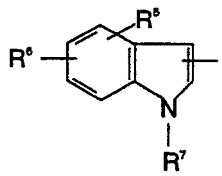

$R^1$, $R^2$ unabhängig voneinander Wasserstoff oder $(C_1-C_4)$Alkyl,

$R^3$ $(C_1-C_4)$Alkoxy-$(C_1-C_4)$alkoxy, Amino-carbonyl-$(C_1-C_4)$alkoxy, $(C_1-C_4)$Alkoxy-$(C_1-C_4)$alkyl-amino-carbonyl-$(C_1-C_4)$alkoxy, Thiazolidinyl-carbonyl-amino, Mono-$(C_1-C_4)$alkylamino-carbonyl-$(C_1-C_4)$alkoxy, $(C_1-C_4)$Alkoxy-carbonyl-$(C_1-C_4)$alkoxy, Hydroxy-carbonyl-$(C_1-C_4)$alkoxy, $(C_1-C_4)$Alkyl-thio, $(C_1-C_4)$Alkylsulfinyl, $(C_1-C_4)$Alkyl-sulfonyl, (2-Oxo-pyrrolidinyl)-$(C_1-C_4)$alkyl-amino-carbonyl-amino, (2-Oxo-piperidinyl)-$(C_1-C_4)$-alkyl-amino-carbonyl-amino, 2-Oxo-pyrrolidinyl, 2-Oxo-piperidinyl, 2,5-Dioxo-piperidinyl, 2,5-Dioxo-pyrrolidinyl, 2-Oxo-imidazolidinyl, 2-Oxo-hexahydro-pyrimidinyl, 2,4-Dioxo-imidazolidinyl, 2,4-Dioxo-hexahydro-pyrimidinyl, 2-Oxo-1,3-oxazolidinyl, 3-Oxo-pyrazolidinyl, (2-($R^9$-carbonyl)-pyrrolidinyl-$(C_1-C_4)$alkoxy, $(C_1-C_4)$Alkyl und $(C_1-C_4)$Alkoxy substituiert durch Pyridyl, Imidazolyl, Oxadiazolyl, Oxo-pyranyl, 2-Hydroxy-pyridinyl, Pyrrolinyl oder Oxo-oxazolidinyl, wobei das Oxo-pyranyl, Oxo-oxazolidinyl und Oxadiazolyl seinerseits durch $(C_1-C_4)$Alkyl oder $(C_1-C_4)$Alkoxy-carbonyl substituiert sein kann, oder einen Rest der Formel $R^{10}$-CO-NH-,

$R^4$ Wasserstoff, $(C_1-C_4)$Alkyl, $(C_1-C_4)$Alkoxy, Hydroxy, $(C_1-C_5)$Alkanoyloxy oder Halogen,

$R^5$, $R^6$, $R^7$ unabhängig voneinander Wasserstoff, $(C_1-C_4)$Alkyl, $(C_1-C_4)$Alkoxy, Hydroxy, Halogen, Amino, Mono$(C_1-C_4)$alkylamino oder Di-$(C_1-C_4)$alkylamino,

$R^8$ Wasserstoff, $(C_1-C_4)$Alkyl oder Phenyl,

$R^9$ Hydroxy, $(C_1-C_4)$Alkoxy, Amino, Mono-$(C_1-C_4)$alkylamino oder Di-$(C_1-C_4)$alkylamino,

$R^{10}$: p-Chlorphenoxymethyl, 3-Pyridyl-oxy-methyl, 3-Pyridyl-methoxy-methyl, 4-Pyridyl-thiomethyl, 4-Pyridyl-sulfinyl-methyl, 4-Pyridyl-sulfonyl-methyl, 2-Oxo-thiazolidin-4-yl, 3-Oxo-perhydro-1,4-thiazin-5-yl, 1-Oxido-3-oxo-perhydro-1,4-thiazin-5-yl, 5-Oxo-perhydro-1,4-thiazepin-3-yl, 1-Oxido-5-oxo-perhydro-1,4-thiazepin-3-yl, 1,1-Dioxido-5-oxo-perhydro-1,4-thiazepin-3-yl, oder einen Rest der Formel

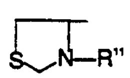 ; 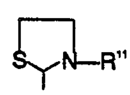

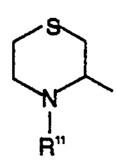 ; 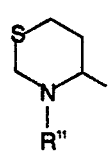

$R^{11}$Wasserstoff oder einen Rest der Formel

$R^{12}$–CO–,

$R^{12}$ Wasserstoff, Alkyl mit 1 bis 5 C-Atomen, Alkoxy mit 1 bis 5 C-Atomen, Benzyl oder Benzyloxy bedeuten, und ihrer pharmakologisch annehmbaren Säureadditionssalze, dadurch gekennzeichnet, daß eine Carbonsäure oder ein Carbonsäurederivat der Formel II

O
||
$R-C-CH-CH_2-X$
|
$R^1$ (II)

worin R und $R^1$ die angegebene Bedeutung besitzen und X = -COOH, -COCl, -CO-O-CO-$R^{13}$, -CO-O$R^{13}$ oder -CN und $R^{13}$ einen organischen Rest bedeuten, mit einem Hydrazin der Formel III

$H_2N-NHR^2$ (III)

worin $R^2$ die angegebene Bedeutung besitzt, umgesetzt und die erhaltene Verbindung gegebenenfalls in ein Säureadditionssalz überführt wird oder daß für den Fall, daß $R^1$ Methyl, $R^2$ Wasserstoff, R einen Rest der Formel

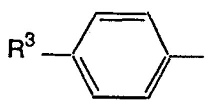

und

$R^3$ einen Rest der Formel

$R^{10}$-CO-NH-

bedeuten, worin $R^{10}$ die bereits genannte Bedeutung besitzt, ein 6-(4-Aminophenyl)-4,5-dihydro-3(2H)-pyridazinon der Formel IV

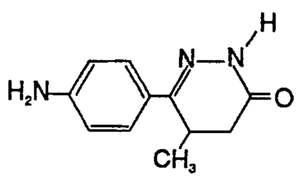

mit einem Acylierungsmittel, das den Acylrest $R^{10}$-CO-einführt, umgesetzt und die entstehende Verbindung gegebenenfalls in ein Säureadditionssalz überführt wird und daß gegebenenfalls eine Verbindung der allgemeinen Formel I mit $R^2$ = H in eine Verbindung der allgemeinen Formel I mit $R^2$ = Alkyl durch Alkylierung mit einem den Alkylrest $R^2$ einführenden Alkylierungsmittel überführt wird.

2. Verfahren nach Anspruch 1, dadurch gekennzeichnet, daß die Ausgangsverbindungen so ausgewählt werden, daß Verbindungen der Formel I entstehen, bei denen

$R^1$, $R^2$ unabhängig voneinander Wasserstoff und/oder ($C_1$-$C_4$)Alkyl,

$R^3$ ($C_1$-$C_4$)Alkoxy-($C_1$-$C_4$)Alkoxy, Amino-carbonyl-($C_1$-$C_4$)alkoxy, Mono-($C_1$-$C_4$)alkylamino-carbonyl-($C_1$-$C_4$)alkoxy, ($C_1$-$C_4$)Alkoxy-carbonyl-($C_1$-$C_4$)alkoxy, ($C_1$-$C_4$)Alkoxy-($C_1$-$C_4$)alkyl-amino-carbonyl-($C_1$-$C_4$)alkoxy, Thiazolidinyl-carbonyl-amino, Hydroxy-carbonyl($C_1$-$C_4$)alkoxy, ($C_1$-$C_4$)Alkyl-thio, ($C_1$-$C_4$)Alkyl-sulfinyl, ($C_1$-$C_4$)Alkyl-sulfonyl, (2-Oxo-pyrrolidin-1-yl)-($C_1$-$C_4$)alkyl-amino-carbonyl-amino, (2-Oxo-piperidin-1-yl)-($C_1$-$C_4$)alkyl-amino-carbonyl-amino, 2-Oxo-pyrrolidin-1-yl, 2-Oxo-piperidin-1-yl, 2,5-Dioxo-pyrrolidin-1-yl, 2-Oxo-imidazolin-1-yl, 2-Oxo-hexahydro-pyrimidin-1-yl, 2,4-Dioxo-imidazolin-1-yl, 2,4-Dioxo-hexahydro-pyrimidin-1-yl, 2-Oxo-1,3-oxazolidin-5-yl, 2-Oxo-1,3-oxazolidin-1-yl, 3-Oxo-pyrazolidin-1-yl, (2-($R^9$-carbonyl)-pyrrolidin-1-yl)-alkoxy, ($C_1$-$C_4$)Alkyl und ($C_1$-$C_4$)Alkoxy substituiert durch Pyridyl, Imidazolyl, Oxadiazolyl, Oxo-pyranyl, 2-Hydroxy-pyridinyl, Pyrrolinyl oder Oxo-oxazolidinyl, wobei das Oxo-pyranyl, Oxo-oxazolidinyl und Oxadiazolyl seinerseits durch ($C_1$-$C_4$)Alkyl oder ($C_1$-$C_4$)Alkoxycarbonyl substituiert sein kann,

$R^4$ Wasserstoff, ($C_1$-$C_4$)Alkyl, ($C_1$-$C_4$)Alkoxy, Hydroxy, Alkanoyloxy mit 1 bis 5 C-Atomen, Chlor, Brom, ($C_1$-$C_4$)Alkyl, ($C_1$-$C_4$)Alkoxy, Hydroxy, Chlor, Brom, Amino, Mono-($C_1$-$C_4$)alkylamino oder Di-($C_1$-$C_4$)alkylamino,

$R^8$ Wasserstoff, ($C_1$-$C_4$)Alkyl oder Phenyl,

$R^9$ Hydroxy, ($C_1$-$C_4$)Alkyl, Amino, Mono-($C_1$-$C_4$)alkylamino oder Di-($C_1$-$C_4$)alkylamino

bedeuten.

3. Verfahren nach Anspruch 1 und/oder 2, dadurch gekennzeichnet, daß die Ausgangsverbindungen so ausgewählt werden, daß Verbindungen der Formel I entstehen, bei denen $R^1$ für Methyl und $R^2$ für Wasserstoff steht und/oder bei denen R Pyrrolyl, Indolyl, 5-Amino-1,3-dimethyl-pyrazol-4-yl, 5-Hydroxy-3-methyl-1-phenyl-pyrazol-4-yl bedeutet.

4. Verfahren nach einem oder mehreren der Ansprüche 1 bis 3, dadurch gekennzeichnet, daß die Ausgangsverbindungen so ausgewählt werden, daß Verbindungen der Formel I entstehen, bei denen R ein durch $R^3$ und $R^4$ substituierter Phenylrest bedeutet und/oder bei denen $R^4$ Wasserstoff, $(C_1$-$C_4)$Alkyl oder $(C_1$-$C_4)$Alkoxy bedeutet.

5. Verfahren I nach einem oder mehreren der Ansprüche 1 bis 4, dadurch gekennzeichnet, daß die Ausgangsverbindungen so ausgewählt werden, daß Verbindungen der Formel I entstehen, bei denen $R^3$ 2-Methoxy-ethoxy, 3-Pyridyl-methoxy, Amino-carbonyl-methoxy, Hydroxy-carbonyl-methoxy, Methylthio, (2-Methoxy-ethyl)-amino-carbonyl-methoxy, 3-Pyridyl-methyl, 5-Methyl-1,3,4-oxadiazol-2-yl, 2-Hydroxy-4-methyl-pyrid-6-yl-methoxy, 2-(Imidazol-1-yl)-ethoxy, (2-Oxo-oxazolidin-5-yl)-methoxy, 2-(2-Oxo-pyrrolidin-1-yl)-ethyl-amino-carbonyl-amino, 2-(Methoxy-carbonyl-pyrrolidin-1-yl)-ethoxy, 2-Oxo-pyrrolidin-1-yl, 2,5-Dioxopyrrolidin-1-yl, 2-Oxo-imidazolidin-1-yl, 3-Oxo-pyrazolidin-1-yl, 2,4-Dioxo-imidazolidin-1-yl, 2-Oxo-oxazolidin-3-yl, Thiazolidin-4-yl-carbonyl-amino bedeutet.

6. Verfahren nach einem oder mehreren der Ansprüche 1 bis 4, dadurch gekennzeichnet, daß die Ausgangsverbindungen so ausgewählt werden, daß Verbindungen der Formel I entstehen, bei denen R ein durch $R^3$ in 4-Stellung substituierter Phenylrest bedeutet.

7. Verfahren nach einem oder mehreren der Ansprüche 1 bis 6, dadurch gekennzeichnet, daß die Ausgangsverbindungen so ausgewählt werden, daß Verbindungen der Formel I entstehen, bei denen $R^{11}$ Wasserstoff, Formyl, Acetyl, tert.Butoxy-carbonyl oder Benzyloxycarbonyl bedeutet.

8. Verfahren nach einem oder mehreren der Ansprüche 1 bis 7, dadurch gekennzeichnet, daß als Acylierungsmittel eine Verbindung der Formel V

$R^{10}$–CO–Y (V)

worin $R^{10}$ die in Anspruch 1 angegebene Bedeutung besitzt und Y = Halogen, Alkyl, insbesondere -Cl oder -Br, -OH, -O-Alkyl, insbesondere mit 1 bis 5 C-Atomen, -O-CO-$R^{10}$ oder -O-CO-O-Alkyl, insbesondere mit 1 bis 5 C-Atomen im Alkylrest, -O-Aryl, -O-Nitroaryl oder -O-Dinitroaryl, insbesondere Phenoxy, 2- oder 4-Nitrophenoxy oder 2,4-Dinitrophenoxy, $-OCH_2CN$ oder den über ein N-Atom gebundenen Rest eines Azols oder Benzazols mit mindestens 2 N-Atomen im quasi-aromatischen Fünfring bedeutet, eingesetzt wird, wobei eine Carbonsäure der Formel Va

$R^{10}$–CO–OH (Va)

vorzugsweise zusammen mit einem Aktivierungsmittel als Acylierungsmittel eingesetzt wird.

9. Verwendung der nach einem oder mehreren der Ansprüche 1 bis 8 herstellbaren substituierten 4,5-Dihydro-3(2H)-pyridazinone der Formel I oder ihrer pharmakologisch annehmbaren Säureadditionssalze für die Herstellung eines Heilmittels zur Bekämpfung und Vorbeugung von Erkrankungen des Herzens und des Kreislaufsystems, einschließlich thromboembolischer Erkrankungen.

**Claims** for the Contracting States: BE, CH, DE, FR, GB, IT, LI, LU, NL, SE

1. Substituted 4,5-dihydro-3(2H)-pyridazinones of the formula I

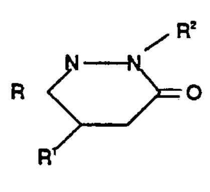

(I)

herein R denotes a radical of the formula

$R^1$ and $R^2$ independently of one another denote hydrogen or $(C_1$-$C_4)$alkyl,

$R^3$: $(C_1$-$C_4)$alkoxy-$(C_1$-$C_4)$alkoxy, amino-carbonyl-$(C_1$-$C_4)$alkoxy, $(C_1$-$C_4)$alkoxy-$(C_1$-$C_4)$alkyl-amino-carbonyl-$(C_1$-$C_4)$alkoxy, thiazolidinyl-carbonyl-amino, mono$(C_1$-$C_4)$alkylamino-carbonyl-$(C_1$-$C_4)$alkoxy, $(C_1$-$C_4)$alkoxycarbonyl-$(C_1$-$C_4)$alkoxy, hydroxy-carbonyl-$(C_1$-$C_4)$alkoxy, $(C_1$-$C_4)$-alkyl-thio, $(C_1$-$C_4)$alkyl-sulphin-

yl, ($C_1$-$C_4$)alkyl-sulphonyl, (2-oxo-pyrrolidinyl)-($C_1$-$C_4$)alkylamino-carbonyl-amino, (2-oxo-piperidinyl)-($C_1$-$C_4$)alkylamino-carbonyl-amino, 2-oxo-pyrrolidinyl, 2-oxo-piperidinyl, 2,5-dioxo-piperidinyl, 2,5-dioxo-pyrrolidinyl, 2-oxo-imidazolidinyl, 2-oxo-hexahydro-pyrimidinyl, 2,4-dioxo-imidazolidinyl, 2,4-dioxo-hexahydro-pyrimidinyl, 2-oxo-1,3-oxazolidinyl, 3-oxo-pyrazolidinyl, (2-($R^9$-carbonyl)-pyrrolidinyl)-($C_1$-$C_4$)alkoxy, ($C_1$-$C_4$)alkyl and ($C_1$-$C_4$)alkoxy substituted by pyridyl, imidazolyl, oxadiazolyl, oxo-pyranyl, 2-hydroxypyridinyl, pyrrolinyl or oxo-oxazolidinyl, it beeing possible for the oxo-pyranyl, oxo-oxazolidinyl and oxadiazolyl in turn to be substituted by ($C_1$-$C_4$)alkyl or ($C_1$-$C_4$)alkoxycarbonyl, or denotes a radical of the formula

$R^{10}$–CO–NH–,

$R^4$ denotes hydrogen, ($C_1$-$C_4$)alkyl, ($C_1$-$C_4$)alkoxy, hydroxyl, ($C_1$-$C_5$)-alkanoyloxy or halogen,
$R^5$, $R^6$ and $R^7$ independently of one another denote hydrogen, ($C_1$-$C_4$)alkyl, ($C_1$-$C_4$)alkoxy, hydroxy, halogen, amino, mono-($C_1$-$C_4$)alkyl-amino or di-($C_1$-$C_4$)alkyl-amino,
$R^8$ denotes hydrogen, ($C_1C_4$)-alkyl or phenyl,
$R^9$ denotes hydroxyl, ($C_1$-$C_4$)alkoxy, amino, mono-($C_1$-$C_4$)alkyl-amino or di-($C_1$-$C_4$)alkyl-amino,
$R^{10}$ denotes p-chloro-phenoxy-methyl, 3-pyridyl-oxy-methyl, 3-pyridyl-methoxy-methyl, 4-pyridyl-thiomethyl, 4-pyridyl-sulphinyl-methyl, 4-pyridyl-sulphonyl-methyl, 2-oxo-thiazolidin-4-yl, 3-oxo-perhydro-1, 4-thiazin-5-yl, 1-oxido-3-oxo-perhydro-1,4-thiazin-5-yl, 5-oxo-perhydro-1,4-thiazepin-3-yl, 1-oxido-5-oxo-perhydro-1,4-thiazepin3-yl, 1,1-dioxido-5-oxo-perhydro-1,4-thiazepin-3-yl, or a radical of the formula

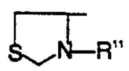 ; 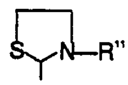

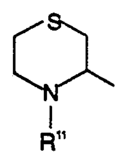 ; 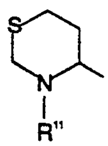

$R^{11}$denotes hydrogen or a radical of the formula

$R^{12}$–CO,

and
$R^{12}$denotes hydrogen, alkyl with 1 to 5 carbon atoms, alkoxy with 1 to 5 carbon atoms, benzyl or benzyloxy, and their pharmacologically acceptable addition salts.

2. Compounds of the formula I according to Claim 1, characterized in that $R^1$ and $R^2$ independently of one another denote hydrogen and/or ($C_1$-$C_4$)alkyl,
$R^3$ denotes ($C_1$-$C_4$)alkoxy-($C_1$-$C_4$)alkoxy, amino-carbonyl($C_1$-$C_4$)alkoxy, mono-($C_1$-$C_4$)alkylamino-carbonyl-($C_1$-$C_4$)alkoxy, ($C_1$-$C_4$)alkoxy-carbonyl-($C_1$-$C_4$)alkoxy, ($C_1$-$C_4$)alkoxy-($C_1$-$C_4$)alkyl-aminocarbonyl-($C_1$-$C_4$)alkoxy, thiazolidinyl-carbonyl-amino, hydroxy-carbonyl-($C_1$-$C_4$)alkoxy, ($C_1$-$C_4$)alkyl-thio, ($C_1$-$C_4$)alkyl-sulphinyl, ($C_1$-$C_4$)alkyl-sulphonyl, (2-oxo-pyrrolidin-1-yl)-($C_1$-$C_4$)alkyl-amino-carbonyl-amino, (2-oxo-piperidin-1-yl)($C_1$-$C_4$)alkyl-amino-carbonyl-amino, 2-oxo-pyrrolidin-1-yl, 2-oxo-piperidin-1-yl, 2,5-dioxo-piperidin-1-yl, 2,5-dioxo-pyrrolidin-1-yl, 2-oxo-imidazolin-1-yl, 2-oxo-hexahydro-pyrimidin-1-yl, 2,4-dioxo-imidazolin-1-yl, 2,4-dioxo-hexahydro-pyrimidin-1-yl, 2-oxo-1,3-oxazolidin-5-yl, 2-oxo-1,3-oxazolidin-1-yl, 3-oxo-pyrazolidin-1-yl, (2($R^9$-carbonyl)-pyrrolidin-1-yl)-alkoxy, ($C_1$-$C_4$)alkyl and ($C_1$-$C_4$)alkoxy, substituted by pyridyl, imidazolyl, oxadiazolyl, oxo-pyranyl, 2-hydroxy-pyridinyl, pyrrolinyl or oxo-oxazolidinyl, it being possible for the oxopyranyl, oxo-oxazolidinyl and oxadiazolyl in turn to be substituted by ($C_1$-$C_4$)alkyl or ($C_1$-$C_4$)alkoxy-carbonyl,
$R^4$ denotes hydrogen, ($C_1$-$C_4$)alkyl, ($C_1$-$C_4$)alkoxy, hydroxyl, alkanoyloxy with 1 to 5 C atoms, chlorine or bromine,
$R^5$, $R^6$ and $R^7$ independently of one another denote hydrogen, ($C_1$-$C_4$)alkyl, ($C_1$-$C_4$)alkoxy, hydroxyl, chlorine, bromine, amino, mono-($C_1$-$C_4$)alkyl-amino or di-($C_1$-$C_4$)alkyl-amino,
$R^8$ denotes hydrogen, ($C_1$-$C_4$)alkyl or phenyl and
$R^9$ denotes hydroxyl, ($C_1$-$C_4$)alkyl, amino, mono-($C_1$-$C_4$)alkyl-amino or di-($C_1$-$C_4$)alkyl-amino.

3. Compounds of the formula I according to Claim 1 and/or 2, characterised in that $R^1$ and $R^2$ independently of one another denote hydrogen or methyl or that $R^1$ represents methyl and $R^2$ represents hydrogen.

4. Compounds of the formula I according to one or several of Claims 1 to 3, characterised in that R denotes pyrrolyl, indolyl, 5-amino-1,3-dimethyl-pyrazol-4-yl, 5-hydroxy-3-methyl-1-phenyl-pyrazol-4-yl or that R is a phenyl radical substituted by $R^3$ and $R^4$, in particular that R is a phenyl radical substituted by $R^3$ in the 4-position.

5. Compounds of the formula I according to one or several of Claims 1 to 4, characterised in that $R^4$ denotes $(C_1\text{-}C_4)$alkyl, $(C_1\text{-}C_4)$alkoxy, in particular that $R^4$ denotes hydrogen.

6. Compounds of the formula I according to one or several of Claims 1 to 5, characterized in that $R^3$ denotes 2-methoxy-ethoxy, 3-pyridyl-methoxy, amino-carbonylmethoxy, hydroxy-carbonyl-methoxy, methylthio, (2-methoxy-ethyl)-amino-carbonyl-methoxy, 3-pyridyl-methyl, 5-methyl-1,3,4-oxadiazol-2-yl, 2-hydroxy-4-methyl-pyrid6-yl-methoxy, 2-(imidazol-1-yl)-ethoxy, (2-oxo-oxazolidin-5-yl)-methoxy, 2-(2-oxo-pyrrolidin-1-yl)-ethyl-aminocarbonyl-amino, 2-(methoxycarbonyl-pyrrolidin-1-yl-ethoxy, 2-oxo-pyrrolidin-1-yl, 2,5-dioxo-pyrrolidin-1-yl, 2-oxo-imidazolidin-1-yl, 3-oxo-pyrazolidin-1-yl, 2,4-dioxo-imidazolidin-1-yl, 2-oxo-oxazolidin-3-yl, thiazolidin-4-yl-carbonyl-amino.

7. Compounds of the formula I according to one or several of Claims 1, 3 and/or 4, characterised in that $R^1$ denotes methyl, $R^2$ denotes hydrogen, R denotes a radical of the formula

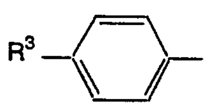

and $R^3$ denotes a radical of the formula

$R^{10}$–CO–NH–

where $R^{10}$ denotes p-chlorophenoxymethyl, 3-pyridyl-oxymethyl, 3-pyridyl-methoxy-methyl, 4-pyridyl-thiomethyl, 4-pyridyl-sulphinyl-methyl, 4-pyridyl-sulphonyl-methyl, 2-oxo-thiazolidin-4-yl, 3-oxo-perhydro-1, 4-thiazin-5-yl, 1-oxido-3-oxo-perhydro-1, 4-thiazin-5-yl, 5-oxo-perhydro1,4-thiazepin-3-yl, 1-oxido-5-oxo-perhydro-1,4-thiazepin3-yl, 1,1-dioxido-5-oxo-perhydro-1,4-thiazepin-3-yl or a radical of the formulae

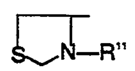 ; 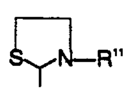

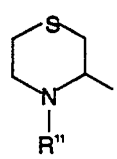 ; 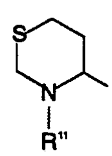

wherein $R^{11}$ has the meaning indicated in Claim 1, and their pharmacologically acceptable addition salts.

8. 4,5-Dihydro-3-(2H)-pyridazinones according to Claim 7, characterised in that $R^{11}$ denotes hydrogen, formyl, acetyl, tert.-butoxy-carbonyl or benzyloxycarbonyl.

9. Process for the preparation of the substituted 4,5dihydro-3(2H)-pyridazinones of the formula I of one or several of Claims 1 to 6, characterised in that a carboxylic acid or a carboxylic acid derivative of the formula II

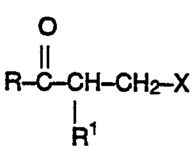 (II)

wherein R and $R^1$ have the meaning given in Claims 1 to 6, X denotes -COOH, -COCl, -CO-O-CO-$R^{13}$, -CO-O$R^{13}$ or -CN and $R^{13}$ denotes an organic radical, is reacted with a hydrazine of the formula III

$$H_2N\text{–}NHR^2 \quad (III)$$

wherein $R^2$ has the meaning given in Claims 1 to 6, and, if appropriate, the resulting compound is converted into an acid addition salt, or that, if appropriate, a compound of the general formula I where $R^2$ = H is converted into a

compound of the general formula I where $R^2$ = alkyl by alkylation with an alkylating agent which introduces the alkyl radical $R^2$, and is then, if appropriate, converted into an acid addition salt.

10. Process for the preparation of the 4,5-dihydro-3(2H)-pyridazinones given in Claim 7 or 8, characterised in that 6-(4-aminophenyl)-4,5-dihydro-3(2H)-pyridazinone of the formula IV

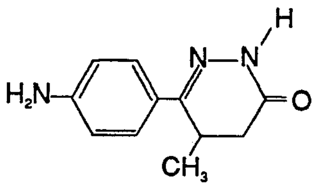

(IV)

is reacted with an acylating agent which introduces the acyl radical $R^{10}$-CO- and, if appropriate, the compound formed is converted into an acid addition salt, the acylating agent employed being preferably a compound of the formula V

$R^{10}$–CO–Y(V)

wherein $R^{10}$ has the meaning given in Claim 7 and Y denotes halogen, in particular -Cl or -Br, -OH, alkyl, -O-alkyl, in particular with 1 to 5 C atoms, -O-CO-$R^{10}$ or -O-CO-O-alkyl, in particular with 1 to 5 C atoms in the alkyl radical, -O-aryl, -O-nitroaryl or -O-dinitroaryl, in particular phenoxy, 2- or 4-nitrophenoxy or 2, 4-dinitrophenoxy, -$OCH_2CN$ or the radical of an azole or benzazole, bonded via an N atom, with at least 2 N atoms in the quasi-aromatic five-membered ring, the carboxylic acid of the formula Va

$R^{10}$–CO–OH(Va)

being preferably used together with an activating agent as acylating agent.

11. Use of the substituted 4,5-dihydro-3-(2H)-pyridazinones of the formula I of one or several of Claims 1 to 8 or their pharmacologically acceptable acid addition salts for preparing a medicine for combating and preventing diseases of the heart and of the circulatory system, including thromboembolic diseases.

12. Pharmaceutical formulation containing an effective dose of a substituted 4,5-dihydro-3(2H)-pyridazinone of the formula I of one or several of Claims 1 to 8 or of a pharmacologically acceptable acid addition salt thereof, in addition to pharmacologically permitted excipients and, if appropriate, additives.

**Claims** for the Contracting State: AT

1. Process for preparing substituted 4,5-dihydro-3(2H)-pyridazinones of the formula I

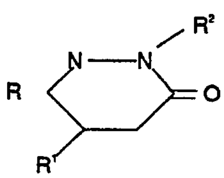

(I)

wherein R denotes a radical of the formula

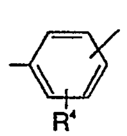

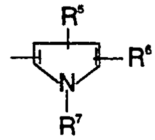

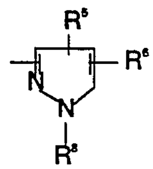

oder

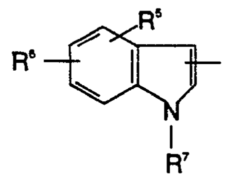

| | |
|---|---|
| $R^1$ and $R^2$ | independently of one another denote hydrogen or ($C_1$-$C_4$)alkyl, |
| $R^3$ | ($C_1$-$C_4$)alkoxy-($C_1$-$C_4$)alkoxy, amino-carbonyl-($C_1$-$C_4$)alkoxy, ($C_1$-$C_4$)alkoxy-($C_1$-$C_4$)alkyl-amino-carbonyl($C_1$-$C_4$)alkoxy, thiazolidinyl-carbonyl-amino, mono-($C_1$-$C_4$)alkylamino-carbonyl-($C_1$-$C_4$)alkoxy, ($C_1$-$C_4$)alkoxy-carbonyl-($C_1$-$C_4$)alkoxy, hydroxy-carbonyl-($C_1$-$C_4$)alkoxy, ($C_1$-$C_4$)-alkyl-thio, ($C_1$-$C_4$)alkyl-sulphinyl, ($C_1$-$C_4$)alkyl-sulphonyl, (2-oxo-pyrrolidinyl)-($C_1$-$C_4$)alkylamino-carbonyl-amino, (2-oxo-piperidinyl)-($C_1$-$C_4$)alkylamino-carbonyl-amino, 2-oxo-pyrrolidinyl, 2-oxo-piperidinyl, 2,5-dioxo-piperidinyl, 2,5-dioxo-pyrrolidinyl, 2-oxo-imidazolidinyl, 2-oxo-hexahydro-pyrimidinyl, 2,4-dioxo-imidazolidinyl, 2,4-dioxo-hexahydro-pyrimidinyl, 2-oxo-1,3-oxazolidinyl, 3-oxo-pyrazolidinyl, (2-($R^9$-carbonyl)-pyrrolidinyl)-($C_1C_4$)alkoxy, ($C_1$-$C_4$)alkyl and ($C_1$-$C_4$)alkoxy substituted by pyridyl, imidazolyl, oxadiazolyl, oxo-pyranyl, 2-hydroxypyridinyl, pyrrolinyl or oxo-oxazolidinyl, it beeing possible for the oxo-pyranyl, oxo-oxazolidinyl and oxa-diazolyl in turn to be substituted by ($C_1$-$C_4$)alkyl or ($C_1$-$C_4$)alkoxycarbonyl, or denotes a radical of the formula |

$R^{10}$–CO–NH–,

| | |
|---|---|
| $R^4$ | denotes hydrogen, ($C_1$-$C_4$)alkyl, ($C_1$-$C_4$)alkoxy, hydroxyl, ($C_1$-$C_5$)-alkanoyloxy or halogen, |
| $R^5$, $R^6$ and $R^7$ | independently of one another denote hydrogen, ($C_1$-$C_4$)alkyl, ($C_1$-$C_4$)alkoxy, hydroxy, halogen, amino, mono-($C_1$-$C_4$)alkyl-amino or di-($C_1$-$C_4$)alkyl-amino, |
| $R^8$ | denotes hydrogen, ($C_1$-$C_4$)-alkyl or phenyl, |
| $R^9$ | denotes hydroxyl, ($C_1$-$C_4$)alkoxy, amino, mono-($C_1$-$C_4$)alkyl-amino or di-($C_1$-$C_4$)alkyl-amino, |
| $R^{10}$ | denotes p-chlorophenoxymethyl, 3-pyridyl-oxy-methyl, 3-pyridyl-methoxy-methyl, 4-pyridyl-thiomethyl, 4-pyridyl-sulphinyl-methyl, 4-pyridyl-sulphonyl-methyl, 2-oxo-thiazolidin-4-yl, 3-oxo-perhydro-1, 4-thiazin-5-yl, 1-oxido-3-oxo-perhydro-1,4-thiazin-5-yl, 5-oxo-perhydro1,4-thiazepin-3-yl, 1-oxido-5-oxo-perhydro-1,4-thiazepin3-yl, 1,1-dioxido-5-oxo-perhydro-1,4-thiazepin-3-yl, or a radical of the formula |

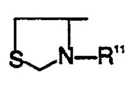

;

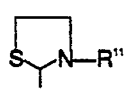

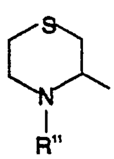

;

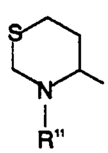

$R^{11}$denotes hydrogen or a radical of the formula

$R^{12}$–CO,

and $R^{12}$ denotes hydrogen, alkyl with 1 to 5 carbon atoms, alkoxy with 1 to 5 carbon atoms, benzyl or benzyloxy, and their pharmacologically acceptable addition salts, characterized in that a carboxylic acid or a carboxylic acid derivative of the formula II

O
||
R–C–CH–$CH_2$–X (II)
|
$R^1$

wherein R and $R^1$ have the meaning indicated and X denotes -COOH, -COCl, -CO-O-CO-$R^{13}$, -CO -O$R^{13}$ or -CN and $R^{13}$ denotes an organic radical, is reacted with a hydrazine of the formula III

$H_2N$–NH$R^2$ (III)

wherein $R^2$ has the meaning indicated and, if appropriate, the resulting compound is converted into an acid addition salt, or that, in case $R^1$ denotes methyl, $R^2$ denotes hydrogen, R denotes a radical of the formula

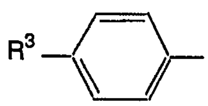

and $R^3$ denotes a radical of the formula

$R^{10}$–CO–NH–

wherein $R^{10}$ has the meaning already mentioned, 6-(4-aminophenyl)-4,5-dihydro-3(2H)-pyridazinone of the formula IV

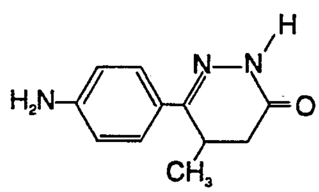

is reacted with an acylating agent which introduces the acyl radical $R^{10}$-CO- and, if appropriate, the compound formed is converted into an acid addition salt, and that, if appropriate a compound of the general formula I wherein $R^2$ denotes H is converted into a compound of the general formula I wherein $R^2$ denotes alkyl by alkylation with an alkylating agent introducing the alkyl radical $R^2$.

2. The process of Claim 1, characterized in that the starting compounds are chosen such that compounds of the formula I are formed wherein $R^1$ and $R^2$ independently of one another denote hydrogen and/or $(C_1$-$C_4)$alkyl,

$R^3$ denotes $(C_1$-$C_4)$alkoxy-$(C_1$-$C_4)$alkoxy, amino-carbonyl$(C_1$-$C_4)$alkoxy, mono-$(C_1$-$C_4)$alkylamino-carbonyl-$(C_1$-$C_4)$alkoxy, $(C_1$-$C_4)$alkoxy-carbonyl-$(C_1$-$C_4)$alkoxy, $(C_1$-$C_4)$alkoxy-$(C_1$-$C_4)$alkyl-aminocarbonyl-$(C_1$-$C_4)$alkoxy, thiazolidinyl-carbonyl-amino, hydroxy-carbonyl-$(C_1$-$C_4)$alkoxy, $(C_1$-$C_4)$alkyl-thio, $(C_1$-$C_4)$alkyl-sulphinyl, $(C_1$-$C_4)$alkylsulphonyl, (2-oxo-pyrrolidin-1-yl)-$(C_1$-$C_4)$alkyl-aminocarbonyl-amino, (2-oxo-piperidin-1-yl)-$(C_1$-$C_4)$alkyl-amino-carbonyl-amino, 2-oxo-pyrrolidin-1-yl, 2-oxo-piperidin-1-yl, 2,5-dioxo-piperidin-1-yl, 2,5-dioxo-pyrrolidin-1-yl, 2-oxo-imidazolin-1-yl, 2-oxo-hexa-hydro-pyrimidin-1-yl, 2,4-dioxo-imidazolin-1-yl, 2,4-dioxo-hexahydro-pyrimidin-1-yl, 2-oxo-1,3-oxazolidin-5-yl, 2-oxo1,3-oxazolidin-1-yl, 3-oxo-pyrazolidin-1-yl, (2-($R^9$-carbonyl)-pyrrolidin-1-yl)-alkoxy, $(C_1$-$C_4)$alkyl and $(C_1$-$C_4)$alkoxy, substituted by pyridyl, imidazolyl, oxadiazolyl, oxo-pyranyl, 2-hydroxy-pyridinyl, pyrrolinyl or oxo-oxazolidinyl, it being possible for the oxopyranyl, oxo-oxazolidinyl and oxadiazolyl in turn to be substituted by $(C_1$-$C_4)$alkyl or $(C_1$-$C_4)$alkoxy-carbonyl,

$R^4$ denotes hydrogen, $(C_1$-$C_4)$alkyl, $(C_1$-$C_4)$alkoxy, hydroxyl, alkanoyloxy with 1 to 5 C atoms, chlorine or bromine,

$R^5$, $R^6$ and $R^7$ independently of one another denote hydrogen, $(C_1$-$C_4)$alkyl, $(C_1$-$C_4)$alkoxy, hydroxyl, chlorine, bromine, amino, mono-$(C_1$-$C_4)$alkyl-amino or di-$(C_1$-$C_4)$alkyl-amino,

$R^8$ denotes hydrogen, $(C_1$-$C_4)$alkyl or phenyl and

$R^9$ denotes hydroxyl, $(C_1$-$C_4)$alkyl, amino, mono-$(C_1$-$C_4)$alkyl-amino or di-$(C_1$-$C_4)$alkyl-amino.

3. The process of Claim 1 and/or 2, characterized in that the starting compounds are chosen such that compounds of formula I are formed wherein $R^1$ represents methyl and $R^2$ represents hydrogen, and/or R denotes pyrrolyl, indolyl, 5-amino-1,3-dimethyl-pyrazol-4-yl,5-hydroxy-3-methyl1-phenyl-pyrazo-4-yl.

4. The process of one or several of Claims 1 to 3, characterized in that the starting compounds are chosen such that R

denotes a phenyl radical substituted by $R^3$ and $R^4$ and/or wherein $R^4$ denotes hydrogen, $(C_1$-$C_4)$alkyl or $(C_1$-$C_4)$alkoxy.

5. The process of the formula I according to one or several of Claims 1 to 4, characterised in that the starting compounds are chosen such that compounds of formula I are formed wherein $R^3$ denotes 2-methoxy-ethoxy, 3-pyridyl-methoxy, amino-carbonyl-methoxy, hydroxy-carbonyl-methoxy, methylthio, (2-methoxy-ethyl)-amino-carbonyl-methoxy, 3-pyridyl-methyl, 5-methyl-1,3,4-oxadiazol-2-yl, 2-hydroxy-4-methyl-pyrid6-yl-methoxy, 2-(imidazol-1-yl)-ethoxy, (2-oxo-oxazolidin-5-yl)-methoxy, 2-(2-oxo-pyrrolidin-1-yl)-ethyl-aminocarbonyl-amino, 2-(methoxycarbonyl-pyrrolidin-1-yl-ethoxy, 2-oxo-pyrrolidin-1-yl, 2,5-dioxo-pyrrolidin-1-yl, 2-oxo-imidazolidin-1-yl, 3-oxo-pyrazolidin-1-yl, 2,4-dioxo-imidazolidin-1-yl, 2-oxo-oxazolidin-3-yl, thiazolidin-4-yl-carbonyl-amino.

6. The process of one or several of Claims 1 to 4, characterized in that the starting compounds are chosen such that compounds of formula I are formed wherein R denotes a phenyl radical substituted by $R^3$ in the 4-position.

7. The process of one or several of Claims 1 to 6, characterized in that the starting compounds are chosen such that $R^{11}$ denotes hydrogen, formyl, acetyl, tert.-butoxy-carbonyl or benzyloxycarbonyl.

8. The process of one or several of Claims 1 to 7, characterized in that the acylating agent employed being a compound of the formula V

$R^{10}$–CO–Y (V)

wherein $R^{10}$ has the meaning given in Claim 1 and Y denotes halogen, in particular -Cl or -Br, -OH, alkyl, -O-alkyl, in particular with 1 to 5 C atoms, -O-CO-$R^{10}$ or -O-CO-O-alkyl, in particular with 1 to 5 C atoms in the alkyl radical, -O-aryl, -O-nitroaryl or -O-dinitroaryl, in particular phenoxy, 2- or 4-nitrophenoxy or 2, 4-dinitrophenoxy, $-OCH_2CN$ or the radical of an azole or benzazole, bonded via an N atom, with at least 2 N atoms in the quasi-aromatic five-membered ring, the carboxylic acid of the formula Va

$R^{10}$–CO–OH (Va)

being preferably used together with an activating agent as acylating agent.

9. Use of the substituted 4,5-dihydro-3-(2H)-pyridazinones of the formula I preparable according to one or several of Claims 1 to 8 or their pharmacologically acceptable acid addition salts for preparing a medicine for combating and preventing diseases of the heart and of the circulatory system, including thromboembolic diseases.

**Revendications** pour les Etats Contractants: BE, CH, DE, FR, GB, IT, LI, LU, NL et SE

1. Dihydro-4,5 *2H* pyridazinones-3 substituées, de formule I

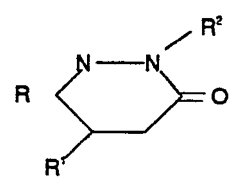

(I)

dans laquelle

R représente un reste de formule

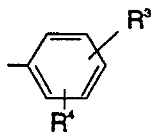

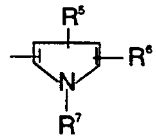

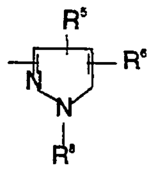

oder

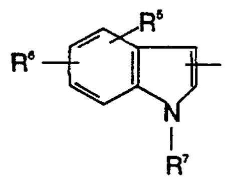

$R^1$ et $R^2$ représentent chacun, indépendamment l'un de l'autre, un atome d'hydrogène ou un groupe alkyle en $C_1$ à $C_4$,

$R^3$ représente un groupe alcoxy (en $C_1$ à $C_4$)-alcoxy (en $C_1$ à $C_4$ amino-carbonyl-alcoxy (en $C_1$ à $C_4$), alcoxy (en $C_1$ à $C_4$)-alkyl (en $C_1$ à $C_4$)-amino-carbonyl-alcoxy (en $C_1$ à $C_4$), thiazolidinyl-carbonyl-amino, monoalkyl amino (en $C_1$ à $C_4$)-carbonyl-alcoxy (en $C_1$ à $C_4$), alcoxy (en $C_1$ à $C_4$)carbonyl-alcoxy (en $C_1$ à $C_4$), hydroxy-carbonyl-alcoxy, (en $C_1$ à $C_4$), alkyl (en $C_1$ à $C_4$)-thio, alkyl (en $C_1$ à $C_4$)-sulfinyle, alkyl (en $C_1$ à $C_4$)-sulfonyle, (oxo-2-pyrrolidinyl)-alkyl (en $C_1$ à $C_4$)-amino-carbonyl-amino, (oxo-2 pipéridinyl)-alkyl (en $C_1$ à $C_4$) amino-carbonyl-amino, oxo-2 pyrrolidinyle, oxo-2 pipéridinyle, dioxo-2,5 pipéridinyle, dioxo-2,5 pyrrolidinyle, oxo-2-imidazolidinyle, oxo-2 hexahydro pyrimidinyle, dioxo-2,4 imidazolidinyle, dioxo-2,4 hexahydro pyrimidinyle, oxo-2 oxazolidinyle-1,3, oxo-3 pyrazolidinyle, (($R^9$-carbonyl)-2 pyrrolidinyl-alcoxy (en $C_1$ à $C_4$), alkyle (en $C_1$ à $C_4$) et alcoxy (en $C_1$ à $C_4$) (substitués par un groupe pyridyle, imidazolyle, oxadiazolyle, oxo-pyrannyle, hydroxy-2-pyridinyle, pyrrolinyle ou oxo-oxazolidinyle, le groupe oxo-pyrannyle, oxo-oxazolidinyle et oxazodiazolyle pouvant, pour sa part, être également substitué par un groupe alkyle en $C_1$ à $C_4$ ou un groupe alcoxy (en $C_1$ à $C_4$)-carbonyle, ou bien un reste de formule

$R^{10}$–CO–NH–,

$R^4$ représente un atome d'hydrogène, un groupe alkyle en $C_1$ à $C_4$, alcoxy en $C_1$ à $C_4$, hydroxyle, alcanoyloxy en $C_1$ à $C_5$ ou halogéno,

$R^5$, $R^6$ et $R^7$ représentent, chacun, indépendamment l'un de l'autre, un atome d'hydrogène, un groupe alkyle en $C_1$ à $C_4$, alcoxy en $C_1$ à $C_4$, hydroxy, halogéno, amino, monoalkyl (en $C_1$ à $C_4$)-amino ou dialkyl (en $C_1$ à $C_4$)-amino,

$R^8$ représente un atome d'hydrogène, un groupe alkyle en $C_1$ à $C_4$ ou phényle,

$R^9$ représente un groupe hydroxyle, alcoxy en $C_1$ à $C_4$, amino, monoalkyl (en $C_1$ à $C_4$) amino ou dialkyl (en $C_1$ à $C_4$) amino,

$R^{10}$ représente un groupe p-chlorophénoxyméthyle, pyridyl-oxy-méthyle-3, pyridyl-méthoxy-méthyle-3, pyridyl-thiométhyle-4, pyridyl-4 sulfinyl-méthyle-4, pyridyl-sulfonyl-méthyle-4, oxo-2 thiazolidinyle-4, oxo-3 perhydro-thiazine-1,4 yle-5, oxydo-1 oxo-3 perhydrothiazine-1,4 yle-5, oxo-5 perhydrothiazépine-1,4 yle-3, oxydo-1 oxo-5 perhydrothiazépine-1,4 yle-3, dioxydo-1,1 oxo-5 perhydrothiazépine1,4 yle-3,ou un reste de formule

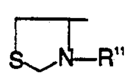 ; 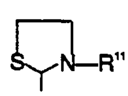

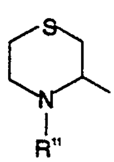 ; 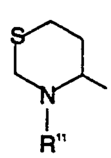

$R^{11}$représente un atome d'hydrogène ou un reste de formule:

$R^{12}$–CO–

$R^{12}$ représente un atome d'hydrogène, un groupe alkyle ayant 1 à 5 atomes de carbone, alcoxy ayant 1 à 5 atomes de carbone, benzyle ou benzyloxy,

et leurs sels d'addition d'acide pharmacologiquement acceptables.

2. Composés de formule I selon la revendication 1, caractérisés en ce que

$R^1$ et $R^2$ représentent chacun, indépendamment l'un de l'autre, un atome d'hydrogène et/ou un groupe alkyle en $C_1$ à $C_4$,

$R^3$ représente un groupe alcoxy (en $C_1$ à $C_4$)-alcoxy (en $C_1$ à $C_4$)-carbonyl-alcoxy (en $C_1$ à $C_4$), alcoxy (en $C_1$ à $C_4$)-alkyle (en $C_1$ à $C_4$)-amino-carbonyl-alcoxy (en $C_1$ à $C_4$), thiazolidinyl-carbonyl-amino, hydroxy-carbonyl-alcoxy (en $C_1$ à $C_4$), alkyl (en $C_1$ à $C_4$)-thio, alkyl (en $C_1$ à $C_4$)-sulfinyle, alkyl (en $C_1$ à $C_4$)-sulfonyle, (oxo-2-pyrrolidinyl)-1-alkyl (en $C_1$ à $C_4$)-amino-carbonyl-amino, (oxo-2 pipéridinyl-1)-alkyl (en $C_1$ à $C_4$) amino-carbonyl-amino, oxo-2 pyrrolidinyle-1, oxo-2 pipéridinyle-1, dioxo-2,5 pipéridinyle-1, dioxo-2,5 pyrrolidinyle-1, oxo-2-imidazolidinyle-1, oxo-2 hexahydro-pyrimidinyle-1, dioxo-2,4 imidazolidinyle-1, dioxo-2,4 hexahydro pyrimidinyle-1, oxo-2 oxazolidinyle-1,3 yle-5, oxo-2 oxalodine-1,3 yle-1, oxo-3 pyrazolidinyle-1, (($R^9$-carbonyl)-pyrrolidinyl-1)-2 alcoxy, alkyle (en $C_1$ à $C_4$) et alcoxy (en $C_1$ à $C_4$) (substitués par un groupe pyridyle, imidazolyle, oxadiazolyle, oxopyrannyle, hydroxy-2-pyridinyle, pyrrolinyle ou oxo-oxazolidinyle, les groupes oxo-pyrannyle, oxo-oxazolidinyle et oxadiazolyle pouvant, de leur côté, être substitués par un groupe alkyle en $C_1$ à $C_4$ ou alcoxy (en $C_1$ à $C_4$)-carbonyle),

$R^4$ représente un atome d'hydrogène, un groupe alkyle en $C_1$ à $C_4$, alcoxy en $C_1$ à $C_4$, hydroxyle, alcanoyloxy ayant 1 à 5 atomes de carbone, ou un atome de chlore ou de brome,

$R^5$, $R^6$ et $R^7$ représentent, chacun, indépendamment l'un de l'autre, un atome d'hydrogène, un groupe alkyle en $C_1$ à $C_4$, alcoxy en $C_1$ à $C_4$, hydroxyle, un atome de chlore ou de brome, un groupe amino, mono-alkyle (en $C_1$ à $C_4$)-amino ou dialkyle en $C_1$ à $C_4$)-amino,

$R^8$ représente un atome d'hydrogène, un groupe alkyle en $C_1$ à $C_4$ ou phényle,

$R^9$ représente un atome d'hydrogène, un groupe alkyle en $C_1$ à $C_4$, amino, monoalkyl (en $C_1$ à $C_4$) amino ou dialkyl (en $C_1$ à $C_4$) amino.

3. Composés de formule I selon la revendication 1 et/ou 2, caractérisés en ce que $R^1$ et $R^2$ représentent chacun, indépendamment l'un de l'autre, un atome d'hydrogène ou un groupe méthyle, ou en ce que $R^1$ représente un groupe méthyle et $R^2$ représente un atome d'hydrogène.

4. Composés de formule I selon une ou plusieurs des revendications 1 à 3, caractérisés en ce que R représente un groupe pyrrolyle, indolyle, amino-5 diméthyl-1,3 pyrazolyle-4, hydroxy-5 méthyl-3 phényl-1 pyrazolyle-4, ou bien en ce que R représente un reste phényle substitué par $R^3$ et $R^4$, et en particulier en ce que R représente un reste phényle substitué en position 4 par $R^3$.

5. Composés de formule I selon une ou plusieurs des revendications 1 à 4, caractérisés en ce que $R^4$ représente un groupe alkyle en $C_1$ à $C_4$, alcoxy en $C_1$ à $C_4$ et en particulier en ce que $R^4$ représente un atome d'hydrogène.

6. Composés de formule I selon une ou plusieurs des revendications 1 à 5, caractérisés en ce que $R^3$ représente un groupe méthoxy-2 éthoxy, pyridyl-3 méthoxy, amino-carbonyl-méthoxy, hydroxy-carbonyl-méthoxy, méthylthio, (méthoxy-2 éthyl)-amino-carbonyl-méthoxy, pyridyl-1 méthyle, méthyl-5 oxadiazole-1,3,4 yle-2, hydroxy-2 méthyl-4 pyridyl-6 méthoxy, (imidazolyl-1)-2 éthoxy, (oxo-2 oxazolidinyl-5)-méthoxy, (oxo-2 pyrrolidinyl-1)-2 éthyl-amino-carbonylamino, (méthoxycarbonyl-pyrrolidinyl-1)-2 éthoxy, oxo-2 pyrrolidinyle-1, dioxo-2,5 pyrrolidinyle-1, oxo-2 imidazolidinyle-1, oxo-3 pyrazolidinyle-1, dioxo-2,4 imidazolidinyle-1, oxo-2 oxazolidinyle-3, thiazolidinyl-4 carbonylamino.

7. Composés de formule I selon une ou plusieurs des revendications 1, 3 et/ou 4, caractérisés en ce que $R^1$ représente un groupe méthyle, $R^2$ représente un atome d'hydrogène, R est un reste de formule

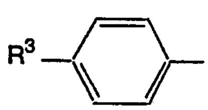

et

$R^3$ est un reste de formule

$R^{10}$–CO–NH–

$R^{10}$ représente un groupe p-chlorophénoxyméthyle, pyridyl-3 oxy-méthyle, pyridyl-3 méthoxy-méthyle, pyridyl-4 thiométhyle, pyridyl-4 sulfinyl-méthyle, pyridyl-4 sulfonyl-méthyl, oxo-2 thiazolidinyle-4, oxo-3 perhydro-thiazine-1,4 yle-5, oxydo-1 oxo-3 perhydrothiazine-1,4 yle-5, oxo-5 perhydrothiazépine-1,4

yle-3, oxydo-1 oxo-5 perhydrothiazépine-1,4 yle-3, dioxydo-1,1 oxo-5 perhydrothiazépine1,4 yle-3, ou un reste de formule

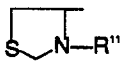 ; 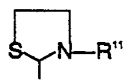

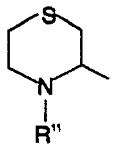 ; 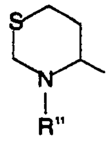

et $R^{11}$ a le sens indiqué à la revendication 1, et leurs sels d'addition d'acide pharmacologiquement acceptables.

8. Dihydro-4,5 *2H* pyridazinones-3 selon la revendication 7, caractérisées en ce que $R^{11}$ représente un atome d'hydrogène, un groupe formyle, acétyle, tertio-butoxycarbonyle ou benzyloxycarbonyle.

9. Procédé pour préparer les dihydro-4,5 *2H* pyridazinones-3 de formule I selon une ou plusieurs des revendications 1 à 6, caractérisé en ce qu'on fait réagir un acide carboxylique ou un dérivé d'acide carboxylique de formule II

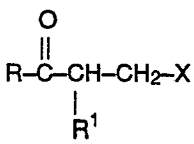 (II)

(dans laquelle R et $R^1$ ont le sens indiqué dans les revendications 1 à 6, et X représente -COOH, -COCl, -CO-O-CO-$R^{13}$, -CO-O$R^{13}$ ou -CH, et $R^{13}$ représente un reste organique) avec une hydrazine de formule III

$N_2N–NHR^2$ (III)

(dans laquelle $R^2$ a le sens indiqué dans les revendications 1 à 6) et l'on transforme éventuellement le composé ainsi obtenu en un sel d'addition d'acide ou bien on transforme éventuellement un composé de formule générale I, dans laquelle $R^2$ représente H, en un composé de formule générale I dans laquelle $R^2$ représente un groupe alkyle, par alkylation à l'aide d'un agent d'alkylation introduisant le reste alkyle $R^2$, puis l'on transforme éventuellement en un sel d'addition d'acide.

10. Procédé pour préparer les dihydro-4,5 *2H* pyridazinones-3 indiquées aux revendications 7 ou 8, caractérisé en ce qu'on fait réagir des (amino-4 phényl-6 dihydro-4,5 *2H* pyridazinones-3 de formule IV

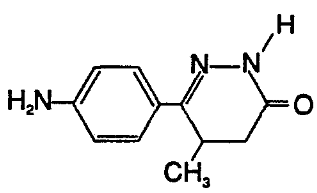 (IV)

avec un agent d'acylation qui introduit le reste acyle $R^{10}$-CO-, et en ce qu'on transforme éventuellement le compose ainsi obtenu en un sel d'addition d'acide, en utilisant comme agent d'acylation avantageusement un composé de formule V

$R^{10}–CO–Y$ (V)

dans laquelle $R^{10}$ a le sens indiqué à la revendication 7 et Y représente un atome d'halogène, en particulier -Cl ou -Br ou bien un groupe -OH, alkyle, -O-alkyle, ayant en particulier 1 à 5 atomes de carbone, -O-CO-$R^{10}$ ou -O-CO-O-alkyle, comportant en particulier 1 à 5 atomes de carbone dans le reste alkyle, -O-aryle, -O-nitroaryle ou -O-dinitroaryle, en particulier phénoxy, nitro-2 ou -4 phénoxy, dinitro2,4 phénoxy, -O-$CH_2$CN ou le reste, fixé par l'intermédiaire d'un

atome de N, d'un azole ou benzazole comportant au moins 2 atomes de N dans un noyau pentagonal quasi-aromatique, l'acide carboxylique de formule Va

$R^{10}$–CO–OH (Va)

étant avantageusement utilisé avec un agent d'activation comme agent d'acylation.

11. Utilisation des dihydro-4,5 *2H* pyridazinones-3 substituées de formule I selon une ou plusieurs des revendications 1 à 8, ou de leurs sels d'addition d'acides pharmacologiquement acceptables, pour la préparation d'un médicament pour lutter contre, et empêcher l'apparition, des maladies du coeur et de l'appareil circulatoire, ce qui comprend des maladies de type thrombose embolique.

12. Préparation pharmaceutique, contenant une dose active d'une dihydro-4,5 *2H* pyridazinone-3 substituée, de formule I, selon une ou plusieurs des revendications 1 à 8, ou d'un sel d'addition d'acide pharmacologiquement acceptable de ce composé, en plus de supports, excipients ou véhicules pharmacologiquement admissibles et éventuellement d'additifs.

**Revendications** pour l'Etat Contractant: AT

1. Procédé pour préparer des dihydro-4,5 *2H* pyridazinones substituées de formule I

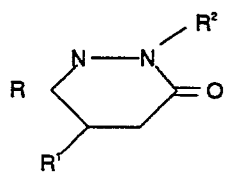

(I)

dans laquelle

R représente un reste de formule

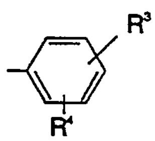

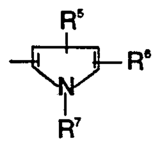

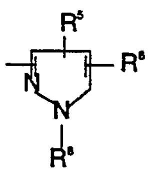

oder

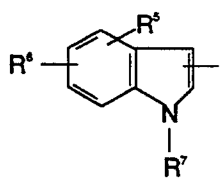

$R^1$ et $R^2$ représentent chacun, indépendamment l'un de l'autre, un atome d'hydrogène ou un groupe alkyle en $C_1$ à $C_4$,

$R^3$ représente un groupe alcoxy (en $C_1$ à $C_4$)-alcoxy (en $C_1$ à $C_4$), amino-carbonyl-alcoxy (en $C_1$ à $C_4$), alcoxy (en $C_1$ à $C_4$)-alkyl (en $C_1$ à $C_4$)-amino-carbonyl-alcoxy (en $C_1$ à $C_4$), thiazolidinyl-carbonyl-amino, monoalkyl amino (en $C_1$ à $C_4$)-carbonyl-alcoxy (en $C_1$ à $C_4$), alcoxy (en $C_1$ à $C_4$)-carbonyl-alcoxy (en $C_1$ à $C_4$), hydroxy-carbonyl-alcoxy, (en $C_1$ à $C_4$), alkyl (en $C_1$ à $C_4$)-thio, alkyl (en $C_1$ à $C_4$)-sulfinyle, alkyl (en $C_1$ à $C_4$)-sulfonyle, (oxo-2-pyrrolidinyl)-alkyl (en $C_1$ à $C_4$)amino-carbonyl-amino, (oxo-2 pipéridinyl)-alkyl (en $C_1$ à $C_4$) amino-carbonyl-amino, oxo-2 pyrrolidinyle, oxo-2 pipéridinyle, dioxo-2,5 pipéridinyle, dioxo-2,5 pyrrolidinyle, oxo-2-imidazolidinyle, oxo-2 hexahydro pyrimidinyle, dioxo-2,4 imidazolidinyle, dioxo-2,4 hexahydro pyrimidinyle, oxo-2 oxazolidinyle-1,3, oxo-3 pyrazolidinyle, (($R^9$-car-

bonyl)-2 pyrrolidinyl-alcoxy (en $C_1$ à $C_4$), alkyle (en $C_1$ à $C_4$) et alcoxy (en $C_1$ à $C_4$) (substitués par un groupe pyridyle, imidazolyle, oxadiazolyle, oxo-pyrranyle, hydroxy-2-pyridinyle, pyrrolinyle ou oxo-oxazolidinyle, le groupe oxo-pyrannyle, oxo-oxazolidinyle et oxazodiazolyle pouvant, pour sa part, être également substitué par un groupe alkyle en $C_1$ à $C_4$ ou un groupe alcoxy (en $C_1$ à $C_4$)-carbonyle, ou bien un reste de formule

$R^{10}$–CO–NH–,

$R^4$ représente un atome d'hydrogène, un groupe alkyle en $C_1$ à $C_4$, alcoxy en $C_1$ à $C_4$, hydroxyle, alcanoyloxy en $C_1$ à $C_5$ ou halogéno,

$R^5$, $R^6$ et $R^7$ représentent, chacun, indépendamment l'un de l'autre, un atome d'hydrogène, un groupe alkyle en $C_1$ à $C_4$, alcoxy en $C_1$ à $C_4$, hydroxy, halogéno, amino, monoalkyl (en $C_1$ à $C_4$)-amino ou dialkyl (en $C_1$ à $C_4$)-amino,

$R^8$ représente un atome d'hydrogène, un groupe alkyle en $C_1$ à $C_4$ ou phényle,

$R^9$ représente un groupe hydroxyle, alcoxy en $C_1$ à $C_4$, amino, monoalkyl (en $C_1$ à $C_4$) amino ou dialkyl (en $C_1$ à $C_4$) amino,

$R^{10}$ représente un groupe p-chlorophénoxyméthyle, pyridyl-oxy-méthyle-3, pyridyl-méthoxy-méthyle-3, pyridylthiométhyle-4, pyridyl-4 sulfinyl-méthyle-4, pyridyl-sulfonyl-méthyle-4, oxo-2 thiazolidinyle-4, oxo-3 perhydro-thiazine-1,4 yle-5, oxydo-1 oxo-3 perhydrothiazine-1,4 yle-5, oxo-5 perhydrothiazépine-1,4 yle-3, oxydo-1 oxo-5 perhydrothiazépine-1,4 yle-3, dioxydo-1,1 oxo-5 perhydrothiazépine1,4 yle-3, ou un reste de formule

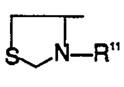 ; 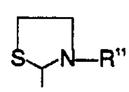

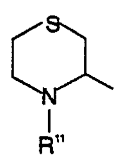 ; 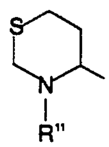

$R^{11}$représente un atome d'hydrogène ou un reste de formule:

$R^{12}$–CO–

$R^{12}$ représente un atome d'hydrogène, un groupe alkyle ayant 1 à 5 atomes de carbone, alcoxy ayant 1 à 5 atomes de carbone, benzyle ou benzyloxy,

et leurs sels d'addition d'acide pharmacologiquement acceptables,

procédé caractérisé en ce qu'on fait réagir un acide carboxylique, ou un dérivé d'acide carboxylique de formule II

$$R-\overset{\overset{\displaystyle O}{\|}}{C}-\underset{\underset{\displaystyle R^1}{|}}{CH}-CH_2-X \qquad (II)$$

(dans laquelle R et $R^1$ ont le sens indiqué, et X représente un groupe -COOH, -COCl, -CO-O-CO-$R^{13}$, -CO-O$R^{13}$ ou -CN, et

$R^{13}$représente un reste organique) avec une hydrazine de formule III

$$H_2N-NHR^2 \qquad (III)$$

(dans laquelle $R^2$ a le sens indiqué) et l'on transforme éventuellement le composé ainsi obtenu en un sel d'addition d'acide ou bien, si $R^1$ représente un groupe méthyle, $R^2$ représente un atome d'hydrogène, R représente un reste de formule

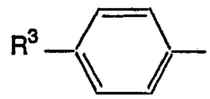

et $R^3$ représente un reste de formule

$R^{10}$–CO–NH–

(dans laquelle $R^{10}$ a le sens déjà indiqué), on fait réagir une (amino-4 phényl)-6 dihydro-4,5 *2H* pyridazinone-3 de formule IV

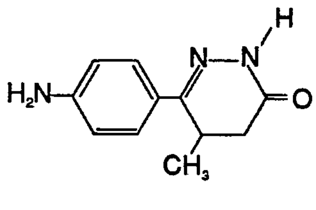

avec un agent d'acylation qui introduit le reste acyle $R^{10}$CO, et l'on transforme éventuellement le composé résultant en un sel d'addition d'acide et l'on transforme éventuellement un composé de formule générale I, dans laquelle $R^2$ représente H, en un composé de formule générale I dans laquelle $R^2$ représente un groupe alkyle, par alkylation à l'aide d'un agent d'alkylation, introduisant le reste alkyle $R^2$.

2. Procédé selon la revendication 1, caractérisé en ce qu'on choisit les composés de départ de manière qu'il en résulte des composés de formule I, dans lesquels:

| | |
|---|---|
| $R^1$ et $R^2$ | représentent chacun, indépendamment l'un de l'autre, un atome d'hydrogène et/ou un groupe alkyle en $C_1$ à $C_4$, |
| $R^3$ | représente un groupe alcoxy (en $C_1$ à $C_4$)-alcoxy (en $C_1$ à $C_4$)-carbonyl-alcoxy (en $C_1$ à $C_4$), alcoxy (en $C_1$ à $C_4$)-alkyle (en $C_1$ à $C_4$)-amino-carbonyl-alcoxy (en $C_1$ à $C_4$), thiazolidinyl-carbonyl-amino, hydroxy-carbonyl-alcoxy (en $C_1$ à $C_4$), alkyl (en $C_1$ à $C_4$)-thio, alkyl (en $C_1$ à $C_4$)-sulfinyle, alkyl (en $C_1$ à $C_4$)-sulfonyle, (oxo-2-pyrrolidinyl)-1-alkyl (en $C_1$ à $C_4$)-amino-carbonyl-amino, (oxo-2 pipéridinyl-1)-alkyl (en $C_1$ à $C_4$) amino-carbonyl-amino oxo-2 pyrrolidinyle-1, oxo-2 pipéridinyle-1, dioxo-2,5 pipéridinyle-1, dioxo-2,5 pyrrolidinyle-1, oxo-2-imidazolidinyle-1, oxo-2 hexahydro-pyrimidinyle-1, dioxo-2,4 imidazolidinyle-1, dioxo-2,4 hexahydro pyrimidinyle-1, oxo-2 oxazolidinyle-1,3 yle-5, oxo-2 oxalodine-1,3 yle-1, oxo-3 pyrazolidinyle-1, (($R^9$-carbonyl)-pyrrolidinyl-1)-2 alcoxy, alkyle (en $C_1$ à $C_4$) et alcoxy (en $C_1$ à $C_4$) (substitués par un groupe pyridyle, imidazolyle, oxadiazolyle, oxopyrannyle, hydroxy-2-pyridinyle, pyrrolinyle ou oxo-oxazolidinyle, les groupes oxo-pyrannyle, oxo-oxazolidinyle et oxadiazolyle pouvant, de leur côté, être substitués par un groupe alkyle en $C_1$ à $C_4$ ou alcoxy (en $C_1$ à $C_4$)-carbonyle), |
| $R^4$ | représente un atome d'hydrogène, un groupe alkyle en $C_1$ à $C_4$, alcoxy en $C_1$ à $C_4$, hydroxyle, alcanoyloxy ayant 1 à 5 atomes de carbone, ou un atome de chlore ou de brome, |
| $R^5$, $R^6$ et $R^7$ | représentent, chacun, indépendamment l'un de l'autre, un atome d'hydrogène, un groupe alkyle en $C_1$ à $C_4$, alcoxy en $C_1$ à $C_4$, hydroxyle, un atome de chlore ou de brome, un groupe amino, mono-alkyle (en $C_1$ à $C_4$)amino ou dialkyle en $C_1$ à $C_4$)-amino, |
| $R^8$ | représente un atome d'hydrogène, un groupe alkyle en $C_1$ à $C_4$ ou phényle, |
| $R^9$ | représente un atome d'hydrogène, un groupe alkyle en $C_1$ à $C_4$, amino, monoalkyl (en $C_1$ à $C_4$) amino ou dialkyl (en $C_1$ à $C_4$) amino. |

3. Procédé selon la revendication 1 et/ou 2, caractérisé en ce qu'on choisit les composés de départ de manière qu'il en résulte des composés de formule I, dans lesquels $R^1$ représente un groupe méthyle et $R^2$ représente un atome d'hydrogène et/ou dans lesquels R représente un groupe pyrrolyle, indolyle, amino-5 diméthyl-1,3 pyrazolyl-4, hydroxy-5 méthyl-3 phényl-1 pyrazolyle-4.

4. Procédé selon une ou plusieurs des revendications 1 à 3, caractérisé en ce qu'on choisit les composés de départ de manière qu'il en résulte des composés de formule I dans lesquels R représente un reste phényle substitué par $R^3$ et $R^4$ et/ou dans lesquels $R^4$ représente un atome d'hydrogène, un groupe alkyle en $C_1$ à $C_4$ ou alcoxy en $C_1$ à $C_4$.

5. Procédé selon une ou plusieurs des revendications 1 à 4, caractérisé en ce qu'on choisit des composés de départ de manière qu'il en résulte des composés de formule I, dans lesquels $R^3$ représente un groupe méthoxy-2 éthoxy, pyridyl-3 méthoxy, amino-carbonyl-méthoxy, hydroxy-carbonyl-méthoxy, méthylthio, (méthoxy-2 éthyl)-amino-carbonyl-méthoxy, pyridyl-1 méthyle, méthyl-5 oxadiazole-1,3,4 yle2, hydroxy-2 méthyl-4 pyridyl-6 méthoxy, (imidazolyl-1)-2 éthoxy, (oxo-2 oxazolidinyl-5)-méthoxy, (oxo-2 pyrrolidinyl-1)-2 éthyl-amino-carbonylamino, (méthoxycarbonyl-pyrrolidinyl-1)-2 éthoxy, oxo-2 pyrrolidinyle-1, dioxo-2,5 pyrrolidinyle-1, oxo-2 imidazolidinyle-1, oxo-3 pyrazolidinyle-1, dioxo-2,4 imidazolidinyle-1, oxo-2 oxazolidinyle-3, thiazolidinyl-4 carbonylamino.

6. Procédé selon une ou plusieurs des revendications 1 à 4, caractérisé en ce qu'on choisit des composés de départ de manière qu'il en résulte des composés de formule I, dans lesquels R représente un reste phényle substitué par $R^3$ en position 4.

7. Procédé selon une ou plusieurs des revendications 1 à 6, caractérisé en ce qu'on choisit les composés de départ de manière qu'il en résulte des composés de formule I, dans lesquels $R^{11}$ représente un atome d'hydrogène, un groupe formule, acétyle, tertio-butoxy-carbonyle ou benzyloxycarbonyle.

8. Procédé selon une ou plusieurs des revendications 1 à 7, caractérisé en ce qu'on utilise comme agent d'acylation un composé de formule V

$$R^{10}\text{–CO–Y} \qquad \text{(V)}$$

dans laquelle $R^{10}$ a le sens indiqué à la revendication 1, et Y représente un atome d'halogène, en particulier -Cl ou -Br, un groupe alkyle, -OH, -O-alkyle ayant en particulier 1 à 5 atomes de carbone, -O-CO-$R^{10}$ ou -O-CO-O-alkyle, ayant en particulier 1 à 5 atomes de carbone dans le reste alkyle, un groupe -O-aryle, -O-nitroaryle ou -O-dinitroaryle, en particulier phénoxy, un groupe nitro-2 ou -4 phénoxy ou bien un groupe dinitro-2,4 phénoxy, -O-$CH_2$CN ou le reste, lié par l'intermédiaire d'un atome de N, d'un azole ou benzazole comportant au moins 2 atomes de N dans un noyau pentagonal quasi-aromatique, en utilisant un acide carboxylique de formule Va

$$R^{10}\text{–CO–OH} \qquad \text{(Va)}$$

avantageusement avec un agent d'activation comme agent d'acylation.

9. Utilisation des dihydro-4,5 *2H* pyridazinones-3 substituées, de formule I, ou de leurs sels d'addition d'acides pharmacologiquement acceptables, pour la préparation d'un médicament destiné à lutter contre, ou à éviter préventivement, des maladies du coeur et de l'appareil circulatoire, ce qui comprend des maladies de type thromboembolique.